# EUROPEAN PATENT APPLICATION

(11) **EP 4 342 984 A1**
(43) Date of publication of application: **27.03.2024**
(21) Application number: 22804747.8
(22) Date of filing: 19.05.2022
(51) Int. Cl.: C12N 5/10, C12Q 1/02, G01N 33/15, G01N 33/50

(54) **METHOD FOR PREDICTING IN VIVO PHARMACOKINETICS OF MOLECULE**

(30) Priority: 19.05.2021 JP 2021084932
(71) Applicant: CHUGAI SEIYAKU KABUSHIKI KAISHA, Tokyo 115-8543 (JP)
(72) Inventor: NOGUCHI, Yuki, Gotemba-shi, Shizuoka 412-8513 (JP); OZEKI, Kazuhisa, Gotemba-shi, Shizuoka 412-8513 (JP); SATO, Kazuki, Gotemba-shi, Shizuoka 412-8513 (JP); NAOI, Sotaro, Gotemba-shi, Shizuoka 412-8513 (JP); TSUTSUI, Haruka, Gotemba-shi, Shizuoka 412-8513 (JP); OHMINATO, Noriaki, Gotemba-shi, Shizuoka 412-8513 (JP)
(74) Representative: Vossius & Partner Patentanwälte Rechtsanwälte mbB
(86) International application number: PCT/JP2022/020814
(87) International publication number: WO 2022/244838

(57) **Abstract**

The present invention provides a method for measuring *in vitro* pharmacokinetics of a molecule, the method including steps of: (a) contacting a molecule with a cell expressing FcRn in an aqueous medium to allow the cell to take up the molecule to be an uptake amount higher than 0.068 pmol/2 × 10⁵ cells, in which step (a) has at least one feature selected from the following (i) to (iii): (i) a period of the contact of the molecule with the cell is 5 hours or more; (ii) the cell after the contact with the molecule is not washed under acidic conditions; and (iii) the cell expresses a target of the molecule on a surface of the cell; and (b) measuring *in vitro* pharmacokinetics of the molecule, in which the molecule contains an FcRn-binding domain, and the like.

## Description

### TECHNICAL FIELD

The present invention relates to a method for measuring *in vitro* pharmacokinetics of a molecule, a method for predicting *in vivo* pharmacokinetics of a molecule, a method for screening a molecule, and the like.

### BACKGROUND ART

Evaluation of pharmacokinetics (PK), which is required in the development of medicaments such as antibody drugs, uses experimental animals such as monkeys and mice. However, it is difficult to evaluate a large number of samples using experimental animals, thus *in vivo* pharmacokinetics is evaluated after narrowing the number of samples in advance. Reducing the number of experimental animals used is also required from an ethical point of view. From these points, the method of predicting *in vivo* pharmacokinetics based on the results of *in vitro* assays has become more important.

Conventionally, as the method for predicting *in vivo* pharmacokinetics, methods utilizing the result *of in vitro* assays using cells have been known (Non Patent Literatures 1 to 3).

Grevys et al. disclose a method for predicting half-life in transgenic mice by using cells (HMEC1-hFcRn) expressing a human neonatal Fc receptor (FcRn) in a human microvascular endothelial cell line (HMEC1) and measuring the efflux amount of IgG antibody to outside of the cells via FcRn *in vitro* by the method named as human endothelial cell-based recycling assay (HERA assay) (Non Patent Literature 1).

Jaramillo et al. disclose that cells expressing human FcRn or rat FcRn in Madin-Darby canine kidney (MDCK) cells were used to measure the activity of the antibodies to permeate the cells via FcRn, i.e., the transcytosis activity, and the measurements were used to rank *in vivo* clearance of the antibodies (Non Patent Literature 2).

Similarly to the method of Jaramillo et al., Chung et al. also disclose that the transcytosis activity was measured using cells expressing human FcRn in MDCK cells, and a correlation was found between the measurement results and *in vivo* clearance in humans (Non Patent Literature 3).

### CITATION LIST

### NON PATENT LITERATURE

[Non Patent Literature 1] Grevys et al, Nat Commun. 2018. Vol. 9: 621
[Non Patent Literature 2] Jaramillo et al, MAbs. 2017. Vol. 9: 781
[Non Patent Literature 3] Chung et al. J Immunol Methods. 2018. Vol. 462: 101

### SUMMARY OF INVENTION

### TECHNICAL PROBLEM

Conventional methods of predicting *in vivo* pharmacokinetics based on the results of *in vitro* assays using cells were not accurate sufficiently.

For example, in the method of Grevys et al., HERA score (R_{X}/R_{WT})/(RA_{X}/RA_{WT}) is calculated from the *in vitro* measurement results, where R represents the amount of protein taken up into the cell within a predetermined period then released outside of the cell, RA represents the remaining amount, X represents the protein of interest (mutant), and WT represents the parent protein used to standardize the results. In this method, the correlation of the HERA score with *in vivo* pharmacokinetics is only observed in the cases where the pharmacokinetic differences between the comparable antibodies are large. Further, it is not disclosed that *in vivo* pharmacokinetics of an antibody having a long blood half-life, such as more than 11 days, is predictable.

In the method of Jaramillo et al, a correlation between the inverse *of in vitro* transcytosis activity (flux) and *in vivo* clearance is observed for Fc-modified antibodies, but when comparing antibodies with small differences in *in vivo* clearance, enough accuracy for predicting *in vivo* clearance has not been obtained from the *in vitro* measurement results (see Figure 6A in Non Patent Literature 2). Further, the results of analysis of antibodies with different antigens shows no correlation between *in vitro* data and *in vivo* data (see Figure 6B in Non Patent Literature 2).

Also in the method of Chung et al., when comparing antibodies with small differences in *in vivo* clearance, enough accuracy for predicting *in vivo* clearance has not been obtained from the *in vitro* measurement results.

Accordingly, the object of the present invention is to provide a method for predicting *in vivo* pharmacokinetics of a molecule based on the measurement results of *in vitro* pharmacokinetics, with higher sensitivity and more accuracy than conventional methods.

### SOLUTION TO PROBLEM

The present inventors have thoroughly investigated the reasons for the insufficient accuracy in predicting *in vivo* pharmacokinetics based on *in vitro* pharmacokinetics in the conventional methods. As a result, the present inventors have found that the prediction accuracy in the conventional method is low due to insufficient cellular uptake of molecules, and further found that increasing the cellular uptake amount of the molecules improves the prediction accuracy. Based on these findings, the present inventors have conducted further studies and completed the present invention.

That is, the present invention provides the following inventions.
[1] A method for measuring *in vitro* pharmacokinetics of a molecule, the method including steps of:
   (a) contacting a molecule with a cell expressing FcRn in an aqueous medium to allow the cell to take up the molecule to be an uptake amount higher than 0.068 pmol/2 × 10⁵ cells, in which step (a) has at least one feature selected from the following (i) to (iii):
      (i) a period of the contact of the molecule with the cell is 5 hours or more;
      (ii) the cell after the contact with the molecule is not washed under acidic conditions; and
      (iii) the cell expresses a target of the molecule on a surface of the cell; and
   (b) measuring *in vitro* pharmacokinetics of the molecule,
      in which the molecule contains an FcRn-binding domain.
[2] The method according to [1], in which the molecule is an antibody containing an FcRn-binding domain and a target-binding domain.
[3] The method according to [1] or [2], in which the cell is a cell transformed to express FcRn.
[4] The method according to any of [1] to [3], in which the cell is a cell transformed to express a target of the molecule on a surface of the cell.
[5] The method according to [3] or [4], in which the cell is a CHO cell, a HEK293 cell, a COS-1 cell, a COS-7 cell, an MDCK cell, an HMEC1 cell, a HELA cell, a HepG2 cell, or a BaF cell.
[6] The method according to [1] or [2], in which the cell is a liver parenchymal cell, a liver non-parenchymal cell, a hepatic sinus endothelial cell, a Kupffer cell, a human umbilical vein endothelial cell, a peripheral blood mononuclear cell PBMC, a macrophage, a mononuclear cell, a B cell, a T cell, a platelet, an NK cell, a neutrophil, an eosinophil, a basophil, a granulocyte, or a dendritic cell.
[7] The method according to any of [1] to [6], in which the cell is allowed to take up the molecule to be an uptake amount higher than 0.10 pmol/2 × 10⁵ cells.
[8] The method according to any of [1] to [7], in which the *in vitro* pharmacokinetics is an efflux amount from inside of a cell to a culture medium, an efflux rate from inside of a cell to a culture medium, a rate of internalization, an amount of transcytosis, a Kp value, a rate of molecular decrease in a cell, a rate of association with FcRn or a target, or a rate of dissociation from FcRn or a target.
[9] The method according to any of [1] to [8], in which FcRn is human FcRn, monkey FcRn, miniature pig FcRn, rat FcRn, mouse FcRn, rabbit FcRn, dog FcRn, or guinea pig FcRn.
[10] The method according to any of [1] to [9], further including a step of (c) calculating an *in vitro* evaluation parameter from a measurement result obtained in step (b).
[11] The method according to [10], in which the *in vitro* evaluation parameter is a clearance index or a HERA score.
[12] The method according to any of [1] to [11], in which the method is used for quality assurance or efficacy prediction of a medicament containing the molecule.
[13] The method according to any of [1] to [12], in which the target of the molecule is a membrane protein.
[14] The method according to [13], in which the target of the molecule is a human IL6 receptor.
[15] A method for predicting *in vivo* pharmacokinetics of a molecule, the method including:
   (a') measuring *in vitro* pharmacokinetics by the method according to any of [1] to [14]; and
   (b') predicting *in vivo* pharmacokinetics of the molecule when administered to a living organism, from a measurement value or an *in vitro* evaluation parameter obtained in step (a').
[16] The method according to [15], in which the *in vivo* pharmacokinetics is a bioavailability, a volume of distribution, a fraction unbound in blood, a clearance, a urinary excretion rate, a blood concentration half-life, or a mean residence time.
[17] The method according to [15] or [16], in which the living organism is a human, a monkey, a miniature pig, a rat, a mouse, a rabbit, a dog, or a guinea pig.
[18] The method according to any of [15] to [17], which is used as an alternative to a pharmacokinetic test method using an animal.
[19] A method for screening a molecule, the method including:
   (a") providing two or more different molecules that bind to the same target,
   (b") measuring *in vitro* pharmacokinetics of each of the two or more molecules provided in step (a") by the method according to any of [1] to [14]; and
   (c") comparing the measurement values or *in vitro* evaluation parameters for each of the two or more molecules obtained in step (b") with each other to select a molecule indicating a desired value.

### ADVANTAGEOUS EFFECTS OF INVENTION

According to the present invention, *in vivo* pharmacokinetics of a molecule can be predicted based on the measurement results of *in vitro* pharmacokinetics, with higher sensitivity and more accuracy than conventional methods. It is thus possible to predict *in vivo* pharmacokinetics of a large number of candidate substances with simplicity and high accuracy in the early stages of the development of medicaments.

The present invention can also contribute to the reduction of the number of experimental animals used, by reducing the number of *in vivo* pharmacokinetic testing.

Furthermore, the present invention can contribute to the development of more pharmacologically effective medicaments by providing an efficient screening method for agents with desired pharmacokinetics.

### BRIEF DESCRIPTION OF DRAWINGS

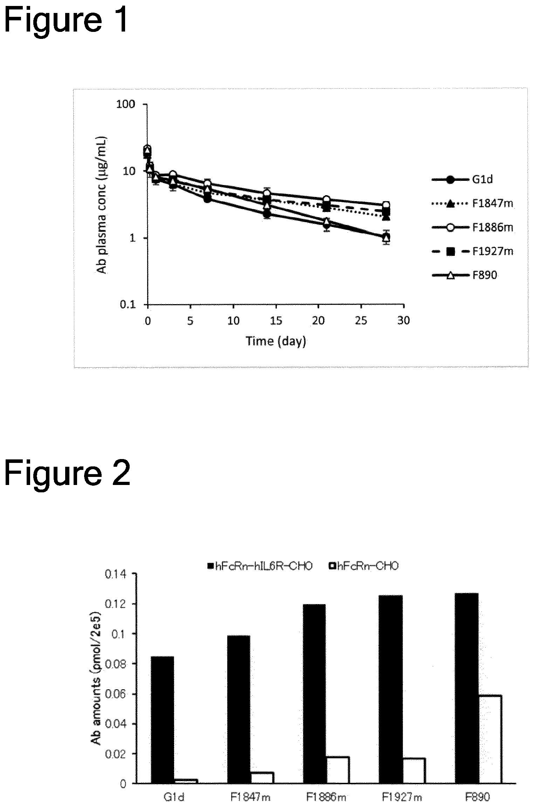

Figure 1 shows the results of mouse plasma pharmacokinetic evaluation of antibodies having different Fc regions (H237-G1d, H237-F1847m, H237-F1886m, H237-F1927m, and H237-F890). To human FcRn transgenic mice (Tg32), 1 mg/kg of each antibody and 1000 mg/kg of Sanglopor were administered. Then, blood sampling was performed by Day 28 and the plasma antibody concentration was measured by ECL assay. The black solid line black circle mark indicates H237-G1d, the black short dash line black triangle mark indicates H237-F1847m, the black solid line white circle mark indicates H237-F1886m, the black long dash line black square mark indicates H237-F1927m, and the black solid line white triangle mark indicates H237-F890.
Figure 2 shows the results of measuring the *in vitro* cellular uptake amount of antibodies having different Fc regions. The cellular uptake amount was shown after hFcRn-hIL6R-CHO cells or hFcRn-CHO cells were allowed to take up each antibody at 37°C for 24 hours and washed with cold 2% FBS-containing PBS.

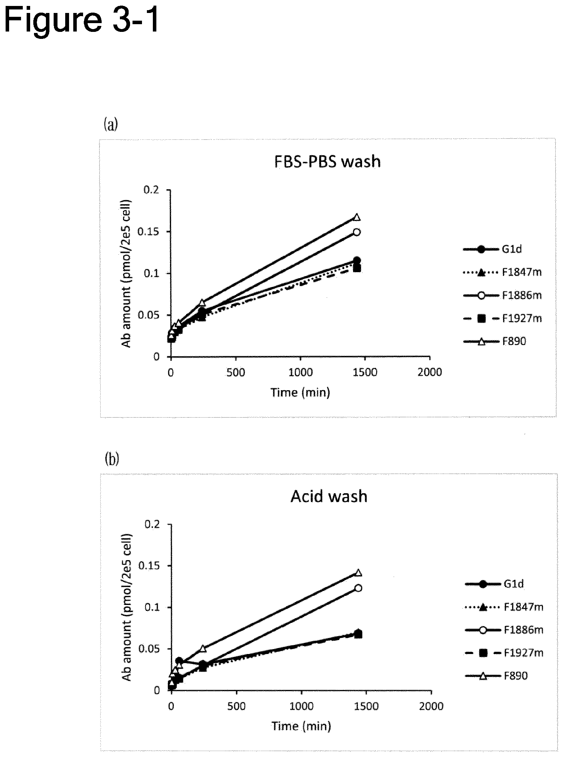

Figure 3-1 shows the results of time-dependent measuring of the cellular uptake of antibodies having different Fc regions. (a) Measurement results when cells were washed with FBS-PBS. The cells were allowed to take up the antibodies, and then washed with FBS-PBS. Both of antibodies bound to the cell surface and internalized antibodies are detected. (b) Measurement results when cells were washed with an acidic culture medium. The cells were allowed to take up the antibodies, and then washed with an acidic culture medium. Antibodies bound to the cell surface are removed and only internalized antibodies are detected. The black solid line black circle mark indicates H237-G1d, the black short dash line black triangle mark indicates H237-F1847m, the black solid line white circle mark indicates H237-F1886m, the black long dash line black square mark indicates H237-F1927m, and the black solid line white triangle mark indicates H237-F890.

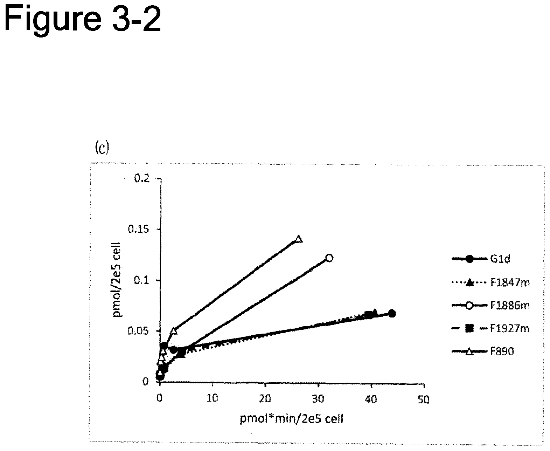

Figure 3-2 shows the results of time-dependent measuring of the cellular uptake of antibodies having different Fc regions. (c) The Integration plot analysis results obtained using the measurement results of (a) and (b) are shown. The black solid line black circle mark indicates H237-G1d, the black short dash line black triangle mark indicates H237-F1847m, the black solid line white circle mark indicates H237-F1886m, the black long dash line black square mark indicates H237-F1927m, and the black solid line white triangle mark indicates H237-F890.

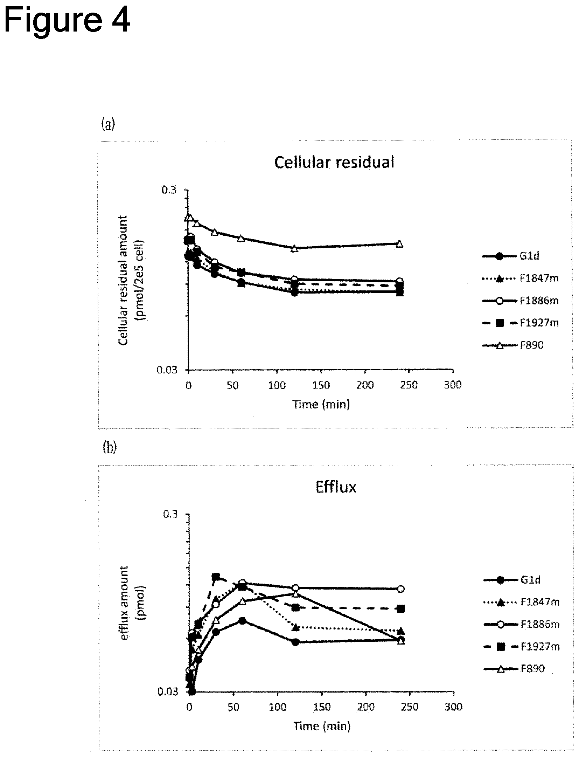

Figure 4 shows the time-dependent changes of the cellular residual amount and the efflux amount into the culture medium of antibodies having different Fc regions. After the cells were allowed to take up each antibody at 37°C for 24 hours, the culture medium was replaced with a fresh one, and the cells were further incubated for 4 hours, then (a) the cellular residual amount and (b) the efflux amount of antibody into the culture medium were measured time-dependently. The black solid line black circle mark indicates H237-G1d, the black short dash line black triangle mark indicates H237-F1847m, the black solid line white circle mark indicates H237-F1886m, the black long dash line black square mark indicates H237-F1927m, and the black solid line white triangle mark indicates H237-F890.

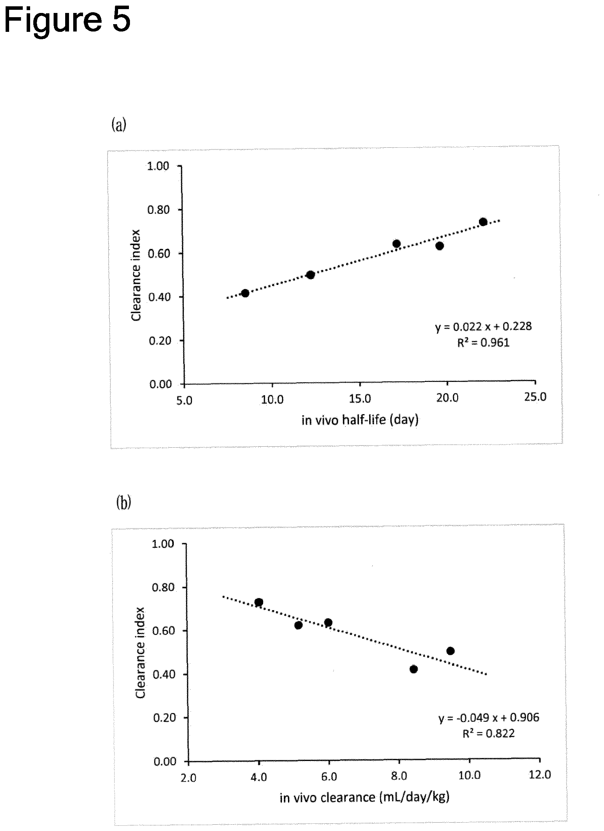

Figure 5 shows the correlation between the clearance index calculated in Example 4 and the plasma half-life (a) or clearance (b) in mice.

### DESCRIPTION OF EMBODIMENTS

Hereinafter, the embodiments of the present invention are described in detail. However, the present invention is not limited thereto, and can be carried out with various modified aspects within the described scope. Unless otherwise noted herein, "A to B" representing a numerical range means "A or more (including A and greater than A) and B or less (including B and less than B)". Unless otherwise specified, "A and/or B" herein means "A, B, or both". In addition, all references cited herein are incorporated herein by reference.

### I. Method for measuring in vitro pharmacokinetics of molecule

The first aspect of the present invention relates to a method for measuring *in vitro* pharmacokinetics of a molecule (hereinafter also referred to as the measuring method of the present invention).

The "*in vivo* pharmacokinetics" as used herein refers to the changes in concentration (amount) of an agent (i.e., the molecule in the present invention) in a living body gone through a series of processes such as absorption, distribution, metabolism, and excretion in the living body after the agent is administered.

After an agent is administered, the processes of absorption, distribution, metabolism, and excretion proceed in parallel in a living body. As the basic pharmacokinetics (PK) parameters to separately describe these processes, (1) bioavailability (F), (2) volume of distribution (Vd or V), (3) fraction unbound in blood (fuB), (4) clearance (CL), and (5) urinary excretion rate (cumulative amount of drug excreted in urine: Ae) have been established (Biometrics Vol. 36, Special Issue, S 3-S 18 (2015)). As indicators of bioavailability, for example, an area under blood concentration-time curve (AUC), a maximum blood concentration (Cmax), and time to reach maximum blood concentration (Tmax) are known. As an indicator of the volume of distribution, for example, volume of distribution at steady state (Vₛₛ) is known. Other known PK parameters include a blood concentration half-life (t_{1/2}), a mean residence time (MRT), an area under the primary moment time curve (AUMC), a vanishing rate constant (kel), a zero time point concentration after administration (C0), and the like.

As used herein, "*in vitro* pharmacokinetics" refers to a behavior of a molecule of interest as measured by contacting the molecule of interest with a cell under artificially constructed conditions *in vitro.* "*In vitro* pharmacokinetics" may be, for example, represented by an efflux amount from inside to outside of a cell, an efflux rate from inside to outside of a cell, a rate of internalization, an amount of transcytosis, a Kp value, a rate of molecular decrease in a cell, a rate of association with FcRn or a target, or a rate of dissociation from FcRn or a target, but are not limited thereto.

The efflux amount from inside to outside of a cell (Efflux amount) is determined by contacting the molecule of interest with the cell for a predetermined period, then exchanging the aqueous medium (e.g., culture medium, buffer, etc.) for one that does not contain the molecule of interest, and then detecting the molecule of interest in the aqueous medium to measure the efflux amount of the molecule of interest from the cell to outside of the cell.

The efflux rate from inside to outside of a cell (Efflux rate) is determined by measuring the Efflux amount of the molecule of interest per unit time.

The rate of internalization is determined by contacting the molecule of interest with the cell for a predetermined period and measuring the amount of the molecule of interest that is taken up into the cell from outside of the cell (e.g., from a culture medium, a buffer, or the like) per unit time. In a preferred aspect, the cells contacted with the molecule of interest for a predetermined period are washed with an aqueous medium that is acidic (less than pH 6.0, e.g., less than or equal to pH 5.5, less than or equal to pH 5.0, less than or equal to pH 4.5, less than or equal to pH 4.0, less than or equal to pH 3.5, or less than or equal to pH 3.0) prior to the measurement of the amount of the molecule of interest, and the molecule of interest that is bound to the surface of the cell is removed. This enables more accurate measurement of the amount of the molecule of interest taken up (internalized) into the cell.

The amount of transcytosis is determined by measuring the amount of permeation from one side to the other side of a cell sheet. For example, the amount of transcytosis can be measured with Transwell(R) systems (Corning Incorporated) and the like (see Non Patent Literatures 2 and 3).

The Kp value is known as a tissue-plasma drug concentration ratio (i.e., the ratio of the concentrations of the molecule of interest between in tissue and in plasma) for *in vivo* pharmacokinetics. However, as used herein, it refers to a ratio of the concentrations of the molecule of interest between in cells and in an aqueous medium (e.g., culture medium) for *in vitro* pharmacokinetics. The Kp value is determined by measuring the amounts of the molecule of interest in cells and in an aqueous medium. As used herein, the Kp value for *in vitro* pharmacokinetics is a value calculated by (amount in cell)/(amount in aqueous medium).

The rate of molecular decrease in a cell is determined by contacting the molecule of interest with the cell for a predetermined period, then exchanging the aqueous medium (e.g., culture medium, buffer, etc.) for one that does not contain the molecule of interest, and then detecting the molecule of interest in the cell to measure the amount of the molecule of interest that is decreased from the cell per unit time.

The rate of association with FcRn or a target is determined by contacting the molecule of interest with the cell for a predetermined short time (e.g., seconds to minutes) and measuring the amount of the molecule of interest bound to FcRn or the target per unit time.

The rate of dissociation from FcRn or a target is determined by contacting the molecule of interest with the cell for a predetermined period (e.g., a time sufficient to reach equilibrium), then exchanging the aqueous medium (e.g., culture medium, buffer, etc.) for one that does not contain the molecule of interest, and then detecting the molecule of interest in the aqueous medium to measure the efflux amount of the molecule of interest into the aqueous medium per unit time. In a preferred embodiment, the contact of the molecule of interest with a cell and the release of the molecule of interest into an aqueous medium can be carried out at a temperature at which the internalization of the molecule of interest into the cell is suppressed (e.g., a temperature at or lower than 4°C).

The "molecule" as used herein (also referred to as the "molecule in the present invention") has the property of being subjected to uptake into the cells used in the measuring method of the present invention and of being subjected to efflux to outside of the cells via a neonatal Fc receptor (FcRn) molecule on the endosome that is an intracellular organelle in the cell. This property depends on the fact the molecule contains an FcRn-binding domain.

FcRn is one of the receptors that recognize the Fc region of an IgG antibody. FcRn is expressed in the fetal placenta and is responsible for transcytosis of IgG from mother to fetus. FcRn is also expressed in cells of an adult such as vascular endothelium, intestinal epithelial cells, and blood cells, and is known to be responsible for exocytosis and transcytosis of IgG and albumin from cells (Nature Reviews Immunology Vol. 7, p. 715-725 (2007)).

Human FcRn is a dimeric protein consisting of a light chain called a β2m subunit and a heavy chain called an alpha subunit having a transmembrane region, the structure of which is similar to that of the major histocompatibility gene complex (MHC) class I molecule. This FcRn dimer is further dimerized and binds to a single molecule IgG (Annual Review of Cell and Developmental Biology Vol. 12, p. 181-220 (1996)). Unlike other IgG antibody Fc receptors, FcRn is known to exhibit pH-dependent binding via electrostatic interactions between anion residues on its own α2 domain and the CH2-CH3 hinge region of IgG (Nature Reviews Immunology Vol. 7, p. 715-725 (2007)).

In the endosome having pH of less than 6.5, IgG taken up into a cell by pinocytosis binds to FcRn with high affinity, escapes degradation at the lysosome and then moves to the surface of the cell under neutral conditions (pH 7.4) where it is dissociated. This pH-dependent binding style allows transcytosis and exocytosis of IgG and contributes to the transport of IgG from mother to fetus and to the prolongation of blood half-life of IgG (approximately 20 days) *in vivo* (Protein Cell Vol. 9(1), p. 15-32 (2018)).

Examples of the FcRn-binding domain include a heavy chain constant region (Fc region) of an antibody and fragments thereof. Other examples of the FcRn binding domain include albumin and a fragment thereof. The binding of albumin to FcRn is described in the literature (J. Exp. Med. (2003) 197 (3), 315-322).

The FcRn binding domain may contain a mutation as long as it can bind to FcRn under a pH environment in the endosome that has a pH of less than 6.5. Examples of the Fc-binding domain containing a mutation include, but are not limited to, mutated Fc regions of the antibodies described in WO 2012/133782 A1, WO 2013/046704 A2, and WO 2017/046994 A1.

The molecule in the present invention may further have a property of binding to a target (i.e., a target binding activity) or a property of catalyzing a reaction in a target (i.e., an enzymatic activity or catalytic activity). The molecule having a target binding activity may function as an agonist or antagonist. A molecule having these properties has a target-binding domain or a catalytic domain. In a preferred embodiment, the molecule in the present invention contains a target-binding domain. This may increase the uptake amount into the cell expressing the target on the surface of the cell.

The "target" as used herein refers to another molecule or structure that binds to the molecule in the present invention, or another molecule or structure that is subjected to a catalytic reaction by the molecule in the present invention. Examples of the "target" include a protein, a nucleic acid, and a sugar chain. The "target" may also be referred to as an antigen, a receptor, a substrate, or the like in relation to the molecule in the present invention.

The target-binding domain is not particularly limited in its structure and the domain of any structure can be used as long as it can bind to the target. Examples of the target-binding domain include an antigen-binding domain of an antibody; Avimer, which contains a module called as an A domain contained in various cell membrane proteins in the living body and consisting of about 35 amino acids (International Publication Nos. WO 2004/044011, WO 2005/040229); Adnectin, which contains a 10Fn3 domain in fibronectin that is a glycoprotein expressed on cell membranes (International Publication No. WO 2002/032925); Affibody, which uses, as a scaffold, an IgG-binding domain consisting of 58 amino acids of Protein A (International Publication No. WO 1995/001937); DARPins (Designed Ankyrin Repeat proteins), which contains, as a scaffold, an ankyrin repeat (AR) that is a repeated sequence of 33 amino acids (International Publication No. WO 2002/020565); Anticalin, which contains, as a scaffold, lipocalin such as neutrophil gelatinase-associated lipocalin (NGAL) (International Publication No. WO 2003/029462); and a variable lymphocyte receptor (VLR) which is a protein that functions in the acquired immune system of j awless species such as lamprey or hagfish and contains a leucine-rich-repeat (LRR) module (International Publication No. WO 2008/016854).

The antigen binding domain herein may be provided from a variable domain of one or more antibodies. Preferably, the antigen binding domain contains an antibody light chain variable region (VL) and an antibody heavy chain variable region (VH). Examples of such an antigen-binding domain preferably include "scFv (single chain Fv)", "single chain antibody", "Fv", "scFv2 (single chain Fv2)", "Fab", or "F(ab')₂".

In certain aspects, the target binding domain contains a variable region of a heavy and/or light chain of an antibody. In a preferred aspect, the target-binding domain contains or consists of variable regions of the heavy and light chains of the antibody.

Examples of the catalytic domain include a catalytic domain of an enzyme.

In a preferred aspect, the molecule in the present invention contains an FcRn-binding domain and a target-binding domain, more preferably containing an Fc region and variable regions of the heavy and light chains of an antibody.

The molecule in the present invention includes medicaments and candidates thereof, and examples thereof include, in addition to proteins such as antibodies described in Examples as molecules, peptide compounds, nucleic acids, toxins, viruses, and DDS formulations such as nanoparticles and microparticles, but are not limited to these as long as they are capable of binding to FcRn. When preparing the molecule in the present invention, the molecule may be prepared by conventional methods using techniques known in the art depending on the type of the molecule. According to the measuring method of the present invention, *in vitro* pharmacokinetics can be measured for these molecules in the same manner as in Examples, and *in vivo* pharmacokinetics thereof can be predicted.

The "protein" as used herein refers to a polymer of amino acids linked via peptide bonds, and may include a peptide compound. The protein may be naturally occurring protein, or non-naturally occurring protein, such as a recombinant protein. Examples of the protein include a cytokine, a bioactive peptide, a biological enzyme, an antibody, and a mutant thereof.

The "antibody" as used herein refers to an immunoglobulin that is naturally occurred or produced through partial or complete synthesis. The antibody can be isolated from a natural resource such as plasma and serum in which the antibody is naturally present and a culture supernatant of hybridoma cells producing the antibody, and can be partially or completely synthesized by using a technique of gene recombination or the like. Preferred examples of the antibody include isotypes of immunoglobulin (i.e., IgG, IgA, IgD, IgE, and IgM) and subclasses of these isotypes. Known as human immunoglobulin are nine subclasses: IgG1, IgG2, IgG3, IgG4, IgA1, IgA2, IgD, IgE, and IgM. In a preferred aspect, the antibody in the measuring method of the present invention is IgG.

The antibody may be either a polyclonal antibody or a monoclonal antibody. In the present invention, a gene recombinant antibody that has been artificially altered to reduce heteroantigenicity or the like, such as a chimeric antibody or a humanized antibody, or a human antibody can be used. The antibody may also be a bispecific antibody. The antibody may be a fragment of an antibody as long as it contains an "FcRn-binding domain". Examples of the fragment of an antibody include an Fc fragment and scFv-CH1-Fc.

The "FcRn-binding domain" of the antibody is not limited as long as it is capable of binding to FcRn, and examples thereof include a heavy chain constant region (Fc region) of the antibody.

The antibody, as a molecule in the present invention, preferably contains an "antigen-binding domain", and more preferably contains variable regions of the heavy and light chains of an antibody. When the cell expresses the antigen on the surface of the cell, this may increase the cellular uptake amount of the molecule in the present invention.

The methods for producing these antibodies are known to those skilled in the art (see, for example, WO 2013/081143).

The structure of an "antigen" herein is not particularly limited as long as it contains an epitope to which an antigen-binding domain binds. The antigen may be inorganic or organic. In some aspects, examples of the antigen include 17-IA, 4-1BB, 4Dc, 6-keto-PGF1a, 8-iso-PGF2a, 8-oxo-dG, A1 adenosine receptor, A33, ACE, ACE-2, activin, activin A, activin AB, activin B, activin C, activin RIA, activin RIA ALK-2, activin RIB ALK-4, activin RIIA, activin RIIB, ADAM, ADAM10, ADAM12, ADAM15, ADAM17/TACE, ADAM8, ADAM9, ADAMTS, ADAMTS4, ADAMTS5, addressin, aFGF, ALCAM, ALK, ALK-1, ALK-7, alpha-1-antitrypsin, alpha-V/beta-1 antagonist, ANG, Ang, APAF-1, APE, APJ, APP, APRIL, AR, ARC, ART, artemin, anti-Id, ASPARTIC, atrial natriuretic factor, av/b3 integrin, Axl, b2M, B7-1, B7-2, B7-H, B-lymphocyte stimulator (BlyS), BACE, BACE-1, Bad, BAFF, BAFF-R, Bag-1, BAK, Bax, BCA-1, BCAM, Bcl, BCMA, BDNF, b-ECGF, bFGF, BID, Bik, BIM, BLC, BL-CAM, BLK, BMP, BMP-2, BMP-2a, BMP-3 osteogenin, BMP-4, BMP-2b, BMP-5, BMP-6, Vgr-1, BMP-7 (OP-1), BMP-8 (BMP-8a, OP-2), BMPR, BMPR-IA (ALK-3), BMPR-IB (ALK-6), BRK-2, RPK-1, BMPR-II (BRK-3), BMP, b-NGF, BOK, bombesin, bone-derived neurotrophic factor, BPDE, BPDE-DNA, BTC, complement factor 3 (C3), C3a, C4, C5, C5a, C10, CA125, CAD-8, calcitonin, cAMP, carcinoembryonic antigen (CEA), cancer-associated antigens, cathepsin A, cathepsin B, cathepsin C/DPPI, cathepsin D, cathepsin E, cathepsin H, cathepsin L, cathepsin O, cathepsin S, cathepsin V, cathepsin X/Z/P, CBL, CCI, CCK2, CCL, CCL1, CCL11, CCL12, CCL13, CCL14, CCL15, CCL16, CCL17, CCL18, CCL19, CCL2, CCL20, CCL21, CCL22, CCL23, CCL24, CCL25, CCL26, CCL27, CCL28, CCL3, CCL4, CCL5, CCL6, CCL7, CCL8, CCL9/10, CCR, CCR1, CCR10, CCR10, CCR2, CCR3, CCR4, CCR5, CCR6, CCR7, CCR8, CCR9, CD1, CD2, CD3, CD3E, CD4, CD5, CD6, CD7, CD8, CD10, CD11a, CD11b, CD11c, CD13, CD14, CD15, CD16, CD18, CD19, CD20, CD21, CD22, CD23, CD25, CD27L, CD28, CD29, CD30, CD30L, CD32, CD33 (p67 protein), CD34, CD38, CD40, CD40L, CD44, CD45, CD46, CD49a, CD52, CD54, CD55, CD56, CD61, CD64, CD66e, CD74, CD80(B7-1), CD89, CD95, CD123, CD137, CD138, CD140a, CD146, CD147, CD148, CD152, CD164, CEACAM5, CFTR, cGMP, CINC, botulinum toxin, Clostridium perfringens toxin, CKb8-1, CLC, CMV, CMV UL, CNTF, CNTN-1, COX, C-Ret, CRG-2, CT-1, CTACK, CTGF, CTLA-4, PD1, PDL1, LAG3, TIM3, galectin-9, CX3CL1, CX3CR1, CXCL, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCR, CXCR1, CXCR2, CXCR3, CXCR4, CXCR5, CXCR6, cytokeratin tumor-associated antigens, DAN, DCC, DcR3, DC-SIGN, Decay accelerating factor, des(1-3)-IGF-I (brain IGF-1), Dhh, digoxin, DNAM-1, Dnase, Dpp, DPPIV/CD26, Dtk, ECAD, EDA, EDA-A1, EDA-A2, EDAR, EGF, EGFR (ErbB-1), EMA, EMMPRIN, ENA, endothelin receptor, enkephalinase, eNOS, Eot, eotaxin 1, EpCAM, ephrin B2/EphB4, EPO, ERCC, E-selectin, ET-1, factor IIa, factor VII, factor VIIIc, factor IX, fibroblast-activating protein (FAP), Fas, FcR1, FEN-1, ferritin, FGF, FGF-19, FGF-2, FGF-3, FGF-8, FGFR, FGFR-3, fibrin, FL, FLIP, Flt-3, Flt-4, follicle-stimulating hormone, fractalkine, FZD1, FZD2, FZD3, FZD4, FZD5, FZD6, FZD7, FZD8, FZD9, FZD10, G250, Gas6, GCP-2, GCSF, GD2, GD3, GDF, GDF-1, GDF-3 (Vgr-2), GDF-5 (BMP-14, CDMP-1), GDF-6 (BMP-13, CDMP-2), GDF-7 (BMP-12, CDMP-3), GDF-8 (myostatin), GDF-9, GDF-15 (MIC-1), GDNF, GDNF, GFAP, GFRa-1, GFR-alpha 1, GFR-alpha 2, GFR-alpha 3, GITR, glucagon, Glut4, glycoprotein IIb/IIIa (GPIIb/IIIa), GM-CSF, gp130, gp72, GRO, growth hormone-releasing factor, hapten (NP-cap or NIP-cap), HB-EGF, HCC, HCMV gB envelope glycoprotein, HCMV gH envelope glycoprotein, HCMV UL, hematopoietic growth factor (HGF), Hep B gp120, heparanase, Her2, Her2/neu (ErbB-2), Her3 (ErbB-3), Her4 (ErbB-4), herpes simplex virus (HSV) gB glycoprotein, HSV gD glycoprotein, HGFA, high-molecular-weight melanoma-associated antigen (HMW-MAA), HIV gp120, HIV IIIB gp120 V3 loop, HLA, HLA-DR, HM1.24, HMFG PEM, HRG, Hrk, human heart myosin, human cytomegalovirus (HCMV), human growth hormone (HGH), HVEM, I-309, IAP, ICAM, ICAM-1, ICAM-3, ICE, ICOS, IFNg, Ig, IgA receptor, IgE, IGF, IGF binding protein, IGF-1R, IGFBP, IGF-I, IGF-II, IL, IL-1, IL-1R, IL-2, IL-2R, IL-4, IL-4R, IL-5, IL-5R, IL-6, IL-6R, IL-8, IL-9, IL-10, IL-12, IL-13, IL-15, IL-18, IL-18R, IL-21, IL-23, IL-27, interferon (INF)-alpha, INF-beta, INF-gamma, inhibin, iNOS, insulin chain A, insulin chain B, insulin-like growth factor 1, integrin alpha 2, integrin alpha 3, integrin alpha 4, integrin alpha 4/beta 1, integrin alpha 4/beta 7, integrin alpha 5 (alpha V), integrin alpha 5/beta 1, integrin alpha 5/beta 3, integrin alpha 6, integrin beta 1, integrin beta 2, interferon gamma, IP-10, I-TAC, JE, kallikrein 2, kallikrein 5, kallikrein 6, kallikrein 11, kallikrein 12, kallikrein 14, kallikrein 15, kallikrein L1, kallikrein L2, kallikrein L3, kallikrein L4, KC, KDR, keratinocyte growth factor (KGF), laminin 5, LAMP, LAP, LAP (TGF-1), latent TGF-1, latent TGF-1 bp1, LBP, LDGF, LECT2, lefty, Lewis-Y antigen, Lewis-Y-related antigen, LFA-1, LFA-3, Lfo, LIF, LIGHT, lipoprotein, LIX, LKN, Lptn, L-selectin, LT-a, LT-b, LTB4, LTBP-1, lung surface, luteinizing hormone, lymphotoxin beta receptor, Mac-1, MAdCAM, MAG, MAP2, MARC, MCAM, MCAM, MCK-2, MCP, M-CSF, MDC, Mer, METALLOPROTEASES, MGDF receptor, MGMT, MHC (HLA-DR), MIF, MIG, MIP, MIP-1-alpha, MK, MMAC1, MMP, MMP-1, MMP-10, MMP-11, MMP-12, MMP-13, MMP-14, MMP-15, MMP-2, MMP-24, MMP-3, MMP-7, MMP-8, MMP-9, MPIF, Mpo, MSK, MSP, mucin (Muc1), MUC18, mullerian-inhibiting substance, Mug, MuSK, NAIP, NAP, NCAD, N-C adherin, NCA 90, NCAM, NCAM, neprilysin, neurotrophin-3, -4, or -6, neurturin, nerve growth factor (NGF), NGFR, NGF-beta, nNOS, NO, NOS, Npn, NRG-3, NT, NTN, OB, OGG1, OPG, OPN, OSM, OX40L, OX40R, p150, p95, PADPr, parathyroid hormone, PARC, PARP, PBR, PBSF, PCAD, P-cadherin, PCNA, PDGF, PDGF, PDK-1, PECAM, PEM, PF4, PGE, PGF, PGI2, PGJ2, PIN, PLA2, placental alkaline phosphatase (PLAP), PIGF, PLP, PP14, proinsulin, prorelaxin, protein C, PS, PSA, PSCA, prostate-specific membrane antigen (PSMA), PTEN, PTHrp, Ptk, PTN, R51, RANK, RANKL, RANTES, RANTES, relaxin A chain, relaxin B chain, renin, respiratory syncytial virus (RSV) F, RSV Fgp, Ret, rheumatoid factor, RLIP76, RPA2, RSK, S100, SCF/KL, SDF-1, SERINE, serum albumin, sFRP-3, Shh, SIGIRR, SK-1, SLAM, SLPI, SMAC, SMDF, SMOH, SOD, SPARC, Stat, STEAP, STEAP-II, TACE, TACI, TAG-72 (tumor-associated glycoprotein-72), TARC, TCA-3, T cell receptor (e.g., T cell receptor alpha/beta), TdT, TECK, TEM1, TEM5, TEM7, TEM8, TERT, testicular PLAP-like alkaline phosphatase, TfR, TGF, TGF-alpha, TGF-beta, TGF-beta Pan Specific, TGF-beta RI (ALK-5), TGF-beta RII, TGF-beta RIIb, TGF-beta RIII, TGF-beta 1, TGF-beta 2, TGF-beta 3, TGF-beta 4, TGF-beta 5, thrombin, thymus Ck-1, thyroid stimulating hormone, Tie, TIMP, TIQ, tissue factor, TMEFF2, Tmpo, TMPRSS2, TNF, TNF-alpha, TNF-alpha beta, TNF-beta 2, TNFc, TNF-RI, TNF-RII, TNFRSF10A (TRAIL R1 Apo-2, DR4), TNFRSF10B (TRAIL R2 DR5, KILLER, TRICK-2A, TRICK-B), TNFRSF10C (TRAIL R3 DcR1, LIT, TRID), TNFRSF10D (TRAIL R4 DcR2, TRUNDD), TNFRSF11A (RANK ODF R, TRANCE R), TNFRSF11B (OPG OCIF, TR1), TNFRSF12 (TWEAK R FN14), TNFRSF13B (TACI), TNFRSF13C (BAFF R), TNFRSF14 (HVEM ATAR, HveA, LIGHT R, TR2), TNFRSF16 (NGFR p75NTR), TNFRSF17 (BCMA), TNFRSF18 (GITR AITR), TNFRSF19 (TROY TAJ, TRADE), TNFRSF19L (RELT), TNFRSF1A (TNF RI CD120a, p55-60), TNFRSF1B (TNF RII CD120b, p75-80), TNFRSF26 (TNFRH3), TNFRSF3 (LTbR TNF RIII, TNFC R), TNFRSF4 (OX40 ACT35, TXGP1 R), TNFRSF5 (CD40 p50), TNFRSF6 (Fas Apo-1, APT1, CD95), TNFRSF6B (DcR3 M68, TR6), TNFRSF7 (CD27), TNFRSF8 (CD30), TNFRSF9 (4-1BB CD137, ILA), TNFRSF21 (DR6), TNFRSF22 (DcTRAIL R2 TNFRH2), TNFRST23 (DcTRAIL R1 TNFRH1), TNFRSF25 (DR3 Apo-3, LARD, TR-3, TRAMP, WSL-1), TNFSF10 (TRAIL Apo-2 ligand, TL2), TNFSF11 (TRANCE/RANK ligand ODF, OPG ligand), TNFSF12 (TWEAK Apo-3 ligand, DR3 ligand), TNFSF13 (APRIL TALL2), TNFSF13B (BAFF BLYS, TALL1, THANK, TNFSF20), TNFSF14 (LIGHT HVEM ligand, LTg), TNFSF15 (TL1A/VEGI), TNFSF18 (GITR ligand AITR ligand, TL6), TNFSF1A (TNF-a Conectin, DIF, TNFSF2), TNFSF1B (TNF-b LTa, TNFSF1), TNFSF3 (LTb TNFC, p33), TNFSF4 (OX40 ligand gp34, TXGP1), TNFSF5 (CD40 ligand CD154, gp39, HIGM1, IMD3, TRAP), TNFSF6 (Fas ligand Apo-1 ligand, APT1 ligand), TNFSF7 (CD27 ligand CD70), TNFSF8 (CD30 ligand CD153), TNFSF9 (4-1BB ligand CD137 ligand), TP-1, t-PA, Tpo, TRAIL, TRAIL R, TRAIL-R1, TRAII,-R2, TRANCE, transferrin receptor, TRF, Trk, TROP-2, TLR (toll-like receptor) 1, TLR2, TLR3, TLR4, TLR5, TLR6, TLR7, TLR8, TLR9, TLR10, TSG, TSLP, tumor-associated antigen CA125, tumor-associated antigen-expressing Lewis-Y-related carbohydrate, TWEAK, TXB2, Ung, uPAR, uPAR-1, urokinase, VCAM, VCAM-1, VECAD, VE-cadherin, VE-cadherin-2, VEFGR-1 (flt-1), VEGF, VEGFR, VEGFR-3 (flt-4), VEGI, VIM, viral antigens, VLA, VLA-1, VLA-4, VNR integrin, von Willebrand factor, WIF-1, WNT1, WNT2, WNT2B/13, WNT3, WNT3A, WNT4, WNT5A, WNT5B, WNT6, WNT7A, WNT7B, WNT8A, WNT8B, WNT9A, WNT9A, WNT9B, WNT10A, WNT10B, WNT11, WNT16, XCL1, XCL2, XCR1, XCR1, XEDAR, XIAP, XPD, HMGB1, IgA, Aβ, CD81, CD97, CD98, DDR1, DKK1, EREG, Hsp90, IL-17/IL-17R, IL-20/IL-20R, oxidized LDL, PCSK9, prekallikrein, RON, TMEM16F, SOD1, chromogranin A, chromogranin B, tau, VAP1, high-molecular-weight kininogen, IL-31, IL-31R, Nav1.1, Nav1.2, Nav1.3, Nav1.4, Nav1.5, Nav1.6, Nav1.7, Nav1.8, Nav1.9, EPCR, C1, C1q, C1r, C1s, C2, C2a, C2b, C3, C3a, C3b, C4, C4a, C4b, C5, C5a, C5b, C6, C7, C8, C9, factor B, factor D, factor H, properdin, sclerostin, fibrinogen, fibrin, prothrombin, thrombin, tissue factor, factor V, factor Va, factor VII, factor VIIa, factor VIII, factor Villa, factor IX, factor IXa, factor X, factor Xa, factor XI, factor Xia, factor XII, factor XIIa, factor XIII, factor XIIIa, TFPI, antithrombin III, EPCR, thrombomodulin, TAPI, tPA, plasminogen, plasmin, PAI-1, PAI-2, GPC3, syndecan-1, syndecan-2, syndecan-3, syndecan-4, LPA, and S1P. In some aspects, examples of the antigen include receptors for hormones and growth factors.

When an antibody such as a bispecific antibody binds to a plurality of epitopes in an antigen molecule, an antigen capable of forming a complex with the antibody may be any of the antigens described above or a combination thereof, in other words, a monomer or a heteromultimer. Non-limiting examples of the heteromultimer include heterodimers such as IL-12 containing IL-12p40 and IL-12p35, IL-23 containing IL-12p40 and IL-23p19 (also referred to as IL-30B), IL-23 containing EBI-3 and IL27p28, and IL-35 containing IL-12p35 and EBI-3.

Examples of the antigen described above include a receptor, and the receptor may be present in a soluble form in a biological fluid such as plasma. Such a soluble receptor is also included in the antigen in the present invention. As a non-limiting aspect of the soluble receptor, an example thereof includes a soluble form IL-6R as described by Mullberg et al. (J. Immunol. (1994) 152 (10), 4958-4968) (e.g., a protein consisting of 1st to 357th amino acids of the IL-6R polypeptide sequence represented by SEQ ID NO: 1 as described in WO 2013/081143).

Examples of the antigen described above include soluble antigens, and the solution in which such a soluble antigen is present is not limited. The soluble antigen may be present in a biological fluid, i.e., in all fluids that fill the vasculature or among tissues/cells in the living body. In a non-limiting aspect, the antigen to which the antibody binds may be present in an extracellular fluid. The extracellular fluid refers to a generic name of plasma, intercellular fluid, lymph, tight connective tissues, cerebrospinal fluid, spinal fluid, aspirates, components in the bone and cartilage such as synovial fluid, alveolar fluid (bronchoalveolar lavage fluid), ascitic fluid, pleural effusion, cardiac effusion, cyst fluid, aqueous humor (hydatoid), or such transcellular fluids (various fluids in glandular cavities resulting from the active transport or secretory activity of cells, and fluids in the lumen of the gut and other body cavities) in vertebrates.

Examples of the antigen described above include soluble antigens and membrane antigens, and it is well known to those skilled in the art to which type each antigen belongs. For example, each antigen can be classified by searching on websites such as UniProtKB (https://www.uniprot.org/) and Human Protein Atlas (https://www.proteinatlas.org/).

When the molecule in the present invention is an antibody, the antibody may preferably be IgG. In certain embodiments, the molecule in the present invention may be an anti-IL-6R antibody, and more particularly a humanized anti-IL-6R antibody.

In the present invention, "nucleic acid" refers to DNA, RNA, and analogs thereof, and may be a natural nucleic acid or a synthetic nucleic acid. Examples of the analog include artificial nucleic acids such as PNA and LNA. The nucleic acid may be single-stranded or double-stranded. The nucleic acid may also be modified. Examples of the modified nucleic acid include those chemically modified at an internucleoside linkage, base, and/or sugar, and those having a modified group at the 5' end and/or 3' end. Examples of the modification to the internucleoside linkage include changes to any of the phosphodiester linkages, phosphorothioate linkages, phosphorodithioate linkages, methylphosphonate linkages, phosphoramidate linkages, non-phosphate linkages, and methylphosphonothioate linkages, or a combination thereof. Examples of the modification to a base include changes to 5-propynyluracil, 2-aminoadenine, and the like. Examples of the modification to sugar include changes to 2'-fluororibose, 2'-O-methyl ribose, and the like.

The nucleic acid is sometimes referred to as siRNA, antisense RNA, miRNA, shRNA, a ribozyme, or an aptamer, depending on their function or use. Examples of the nucleic acid used in the present invention also include CpG oligonucleotides that act on Toll-like receptor 9 (TLR9) to activate innate immunity.

The base length of the nucleic acid is not limited as long as it can be taken up into the cell via Stabilin, and examples thereof include the range of 4 to 100 base lengths, 10 to 50 base lengths, 10 to 40 base lengths, or 10 to 30 base lengths.

In one embodiment, when the molecule in the present invention is a nucleic acid, the target (or receptor) thereof may be Stabilin.

As used herein, Stabilin refers to a protein belonging to a family of transmembrane proteins known as nucleic acid receptors. In mammals, two homologs of Stabilin-1 and Stabilin-2 are known, and Stabilin in the present invention may be any of them. In humans, Stabilin-1 (NCBI accession number: NP_055951.2) and Stabilin-2 (NCBI accession number: NP_060034.9) are known and they have been reported to be expressed in LSEC, spleen, adrenal cortex, lymph nodes, and sinusoidal macrophages.

The "peptide compound" as used herein refers to a compound formed by amide bonding or ester bonding of amino acids or amino acid analogs. The molecular form of the peptide compound can be linear, cyclic, or cyclic having a linear portion.

The number of amide bonds or ester bonds (the number or length of amino acids or amino acid analogs) is not particularly limited, but when the molecular form is cyclic having a linear portion, it is preferably within 30 residues in a total of the cyclic portion and linear portion. It is more preferable that the total number of amino acids in a total of the cyclic portion and linear portion is 13 residues or less. To obtain high metabolic stability, it is more preferable that the total number of amino acids is 9 or more. In addition to the above, the number of amino acids and amino acid analogs constituting the cyclic portion is preferably 5 to 12. Furthermore, the number of amino acids and amino acid analogs constituting the cyclic portion is more preferably 5 to 11, further preferably 7 to 11 residues, in addition to the description above. In particular, 9 to 11 residues are preferred. The number of amino acids and amino acid analogs (number of units) in the linear portion is preferably 0 to 8, further preferably 0 to 3. Unless otherwise specified in the present application, the amino acids may include amino acid analogs.

As used herein, the "amino acid" and "amino acid analog" constituting the peptide compound may be referred to as "amino acid residue" and "amino acid analog residue", respectively.

The amino acid is α-, β-, or γ-amino acid, and is not limited to a naturally occurring amino acid and may be a non-naturally occurring amino acid. The naturally occurring amino acid in the present application refers to 20 amino acids contained in a protein, specifically, Gly, Ala, Ser, Thr, Val, Leu, Ile, Phe, Tyr, Trp, His, Glu, Asp, Gln, Asn, Cys, Met, Lys, Arg, and Pro. When the amino acid is α-amino acid, it may be either L-amino acid or D-amino acid, or α,α-dialkylamino acid. The selection of an amino acid side chain is not particularly limited, and the side chain is freely selected from, in addition to a hydrogen atom, an alkyl group, an alkenyl group, an alkynyl group, an aryl group, a heteroaryl group, an aralkyl group, a cycloalkyl group, and the like. A substituent may be added to each of the groups, and these substituents are freely selected, for example, from any functional groups including an N atom, an O atom, an S atom, a B atom, a Si atom, and a P atom (i.e., an alkyl group, alkenyl group, alkynyl group, aryl group, heteroaryl group, aralkyl group, cycloalkyl group, and the like that may be substituted).

The "amino acid" and "amino acid analog" constituting a peptide compound include all isotopes corresponding to each. The isotope of "amino acid" or "amino acid analog" is one in which at least one atom is substituted with an atom having the same atomic number (the number of protons) and a different mass number (the sum of numbers of protons and neutrons). Examples of the isotope contained in the "amino acid" or "amino acid analog" constituting a peptide compound herein include a hydrogen atom, a carbon atom, a nitrogen atom, an oxygen atom, a phosphorus atom, a sulfur atom, a fluorine atom, a chlorine atom, and the like, and they include ²H, ³H; ¹³C, ¹⁴C; ¹⁵N; ¹⁷O, ¹⁸O; ³¹P, ³²P; ³⁵S; ¹⁸F; ³⁶Cl. and the like, respectively.

When the fluorescent labeling kit Alexa Fluor(R) 488 Protein Labeling Kit (Invitrogen) is used for detecting a peptide compound, it is desirable that the peptide compound has an amino acid having an amino group. Examples of such an amino acid include lysine (Lys). In addition, amino acids having a thiol group can also be labeled with a thiol-reactive fluorescent dye. Examples of such an amino acid include cysteine (Cys).

When the molecule in the present invention is a peptide compound, the target (or receptor) thereof is preferably PEPT1 or PEPT2.

Nanoparticles and microparticles are known to be used in formulations for drug delivery systems, commonly called DDS. Examples thereof include, but are not limited to, liposomes, micelles, dendrimers, nanoemulsions, iron nanoparticles, gold nanoparticles, and PLGA particles (Organ Biology Vol. 24 No. 1 2017, 54-60).

In a preferred embodiment, the molecule in the present invention includes nanoparticles and microparticles conjugated to a molecule that specifically binds to a specific cell. For example, an antigen-binding molecule against a surface antigen of the cell can be conjugated to these particles. In another example, a molecule containing an FcRn-binding domain can be conjugated to these particles. In one embodiment, the molecule in the present invention may be a nanoparticle/microparticle conjugated to an antibody containing an FcRn-binding domain and/or a target-binding domain.

In the present invention, the "toxin" is not particularly limited as long as it can specifically deliver a cytotoxic agent, toxin, or radioisotope to particular cells, and damage them. For example, a molecule in which a molecule that specifically binds to the cell (e.g., an antigen-binding molecule to a cell surface antigen of the cell) is conjugated to a cytotoxic agent, a toxin, or a radioisotope can be produced. Examples of the "molecule that specifically binds to a cell" include antibodies, nucleic acids, and peptide compounds described above. Such molecules can be used to efficiently deliver a cytotoxic agent, a toxin, or a radioisotope to the cell. As a result, the cell can be specifically damaged.

Examples of the cytotoxic agent include maytansinoids (see U.S. Patent Nos. 5,208,020 and 5,416,064, and European Patent No. 0,425,235 B1); auristatins such as monomethyl auristatin drug moieties DE and DF (MMAE and MMAF) (see U.S. Patent Nos. 5,635,483, 5,780,588 and 7,498,298); dolastatins; calicheamicin or derivatives thereof (see U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); anthracyclines such as daunomycin or doxorubicin (see Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; taxanes such as docetaxel, paclitaxel, larotaxel, tesetaxel, and ortataxel; trichothecenes; and CC1065.

Examples of the toxin include, but are not limited to, enzymatically active toxins or fragments thereof including the following: diphtheria-A chain, non-binding active fragments of diphtheria toxins, exotoxin A chain (from Pseudomonas aeruginosa), ricin A chain, abrin A chain, modeccin A chain, alpha-sarcin, Aleurites fordii proteins, dianthin proteins, Phytolacca americana proteins (PAPI, PAPII, and PAP-S), momordica charantia inhibitor, curcin, crotin, saponaria officinalis inhibitor, gelonin, mitogellin, restrictocin, phenomycin, enomycin, and trichothecenes.

Examples of the radioisotope include ²¹¹At, ¹³¹I, ¹²⁵I, ⁹⁰Y, ¹⁸⁶Re, ¹⁸⁸Re, ¹⁵³Sm, ²¹²Bi, ³²P, ²¹²Pb, and radioisotopes of Lu.

As the molecule in the present invention, a virus can also be used. For example, according to the measuring method of the present invention, *in vitro* kinetics of a virus or a viral protein or a portion thereof shown below can be measured. Examples of the virus include viruses used in gene therapy, such as retroviruses, adenoviruses, adeno-associated viruses, herpes simplex viruses, lentiviruses, pox viruses, and Epstein-Barr viruses (Adv Biomed Res. (2012) 1:27. doi: 10.4103/2277-9175.98152), and drug delivery viruses such as the Red clover necrotic mosaic virus (RCNMV) (Methods Mol Biol. 2011; 726: 207-221). Examples of the viral protein or a portion thereof include partial peptides of HIV-1 tat protein, L2 peptides of human papillomavirus, and envelope L proteins of HBV.

In one aspect, the measuring method of the present invention includes steps of:
(a) contacting a molecule with a cell expressing FcRn in an aqueous medium to allow the cell to take up the molecule to be an uptake amount higher than 0.068 pmol/2 × 10⁵ cells; and
(b) measuring *in vitro* pharmacokinetics of the molecule.

In the measuring method of the present invention, step (b) is not necessarily to be started after the completion of step (a). That is, step (b) may be started after the cellular uptake of the molecule in the present invention has been completed in step (a), or may be started when the molecule in the present invention is put in a state that may provide the cellular uptake of the molecule in step (a).

The cell used in the measuring method of the present invention is not particularly limited as long as the cell can be contacted with a molecule of interest *in vitro* and express FcRn, and examples thereof include a cell harvested from a living organism, a primary cultured cell, or a strain cell. Expression of FcRn is confirmed by staining cells with fluorescently labeled anti-FcRn antibodies and measuring the fluorescence by FACS to confirm that its histogram is shifted to a higher fluorescence intensity side than that of a control antibody (e.g., isotype-controlled antibody). More quantitative assessment may be performed by analyzing cells with a liquid chromatography-mass spectrometer (LC-MS) and measuring the amount of FcRn-derived peptides.

In a preferred aspect, FcRn may be FcRn of the species for which *in vivo* pharmacokinetics is desired to be predicted, and examples thereof include human FcRn, monkey FcRn, miniature pig FcRn, rat FcRn, mouse FcRn, rabbit FcRn, dog FcRn, or guinea pig FcRn, hamster FcRn, chimpanzee FcRn, marmoset FcRn, ferret FcRn, or cat FcRn.

In one embodiment, the cell can be a cell transformed to express FcRn. Such transformation can be performed, for example, by introducing a polynucleotide encoding FcRn into the cell. As a promoter, a common promoter used for expression in animal cells can be used, and examples thereof include CMV, PGK, RSV, CAG, EF-1 alpha, SV40, TRE, Oct3/4, and Nanog (PLoS One. 2010; 5(5): e10611). By using such a promoter, a sufficient amount of FcRn can be expressed.

In one embodiment, the cell can be a cell transformed to express a target of the molecule in the present invention at the surface of the cell. When the target is a protein, such transformation can be performed by introducing a polynucleotide encoding the protein into the cell. The greater the expression amount of the target, the greater the cellular uptake amount of the molecule in the present invention can be. Here, the same promoter used when expressing FcRn can be used as a promoter, and using such a promoter, a sufficient amount of the target can be expressed.

The cell used for producing the transformed cell described above is not particularly limited as long as the cell is applicable to transformation techniques that introduce foreign genes into the cell, such as transfection or transduction. Examples of such a cell include a CHO cell, a HEK293 cell, a COS-1 cell, a COS-7 cell, an MDCK cell, an HMEC1 cell, a HELA cell, a HepG2 cell, or a BaF cell, and in certain embodiments, the cell may be a CHO cell.

In one embodiment, the cell may be a cell expressing endogenous FcRn, i.e., a cell expressing FcRn without being subjected to any manipulation for over-expressing exogenous FcRn. Examples of such a cell include a liver parenchymal cell, a liver non-parenchymal cell, a hepatic sinus endothelial cell, a Kupffer cell, a human umbilical vein endothelial cell, a peripheral blood mononuclear cell PBMC, a macrophage, a mononuclear cell, a B cell, a T cell, a platelet, an NK cell, a neutrophil, an eosinophil, a basophil, a granulocyte, or a dendritic cell.

In one embodiment, the cell may be a cell expressing an endogenous protein that is a target of the molecule in the present invention on the surface of the cell, i.e., a cell expressing a target protein on the surface of the cell without being subjected to any manipulation for over-expressing an exogenous target protein. Such a cell may be suitably selected depending on the target protein.

In one embodiment, a cell or cell line with high endocytosis activity may be used as the cell. Using such a cell, the cellular uptake amount of the molecule in the present invention may be increased. Examples of such a cell or cell line include phagocytes such as a macrophage, a neutrophil, an eosinophil, and a monocyte, and a strain cell thereof. Phagocytes have strong phagocytosis and high uptake ability. The macrophage include, for example, a liver Kupffer cell, an alveolar macrophage, brain microglia.

In a preferred embodiment, the cell is a cell transformed to express FcRn, may be more preferably a cell transformed to express FcRn and transformed to express a target of the molecule in the present invention on the surface of the cell.

The "aqueous medium" as used herein means a liquid in which water is an essential component. The aqueous medium is not particularly limited as long as the cellular function of the cell used in the measuring method of the present invention, such as endocytosis, is not lost, and the molecule in the present invention can be stably present therein. Examples of the aqueous medium include a buffer such as phosphate buffered saline (PBS) and a liquid culture medium such as Dulbecco's Modified Eagle Medium (DMEM). In a preferred embodiment, the aqueous medium may be a liquid culture medium from the viewpoint of reducing the load on the cell.

In one embodiment of step (a), the cellular uptake of the molecule in the present invention can be performed by contacting the molecule with a cell in an aqueous medium under conditions in which the cell used does not lose its cellular function, such as endocytosis. Such conditions can be appropriately set depending on the cell used. For example, when a mammalian-derived cell such as a CHO cell is used, incubation may be carried out at 30 to 40°C, preferably 36 to 38°C, in a liquid culture medium.

In a particular embodiment of step (a), the cellular uptake of the molecule in the present invention can be performed by contacting a molecule of interest with a cell in an aqueous medium at a temperature at which the internalization of the molecule into the cell is suppressed (e.g., a temperature at or lower than 4°C). This enables the molecule attached to the surface of the cell but not internalized into the cell to be a target to be measured, and a more accurate measurement of, for example, the rate of dissociation from FcRn or a target can be achieved.

The cellular uptake of the molecule in the present invention is carried out so that the uptake amount is higher than 0.068 pmol/2 × 10⁵ cells. The measurement of the uptake amount is performed by contacting the molecule in the present invention with a cell for a predetermined period depending on each *in vitro* pharmacokinetics to be measured, then taking off the aqueous medium containing the molecule not taken up into the cell, and measuring the amount of the molecule that is internalized into the cell and/or the molecule bound to the surface of the cell. Measurements of the uptake amount can be performed using appropriate measurement means depending on the molecule, and examples thereof include a measurement means using a label such as a fluorescent dye, a measurement means using an antibody against the molecule such as Enzyme-Linked Immuno Sorbent Assay (ELISA) method, and a measurement means for quantifying the molecule or a fragment thereof by a liquid chromatography-mass spectrometer (LC-MS). When the ELISA method or the measurement method with LC-MS is used, the measurement may be performed by solubilizing the cell and quantifying the concentration of the molecule contained in the cell lysate, and the measurement can be performed by a routine procedure using techniques commonly used in the art.

In one embodiment, the measurement of the uptake amount may be performed using a label added to the molecule in the present invention. For example, when the molecule in the present invention is a protein, a label such as a fluorescent dye may be added to the protein, and the label may be used to measure the amount of the protein. Methods of labeling the protein are not limited to specific methods and can be performed by a routine procedure using techniques commonly used in the art. Examples of the method of labeling protein include fluorescent labeling, biotin labeling, peptidic tag labeling (His tag, flag tag, HA tag, or the like), gold colloid labeling, magnetic bead labeling, radioisotope (RI) labeling, and enzyme labeling (with Horse Radish Peroxydase (HRP), Alkaline Phosphatase (AP) or the like). Examples of commonly used-fluorescent labeling include Rhodamin, VioBlue, DyLight 405, DY-405, Alexa Fluor 405, AMCA, AMCA-X, Pacific Blue, DY-415, Royal Blue, ATTO 425, Cy2, ATTO 465, DY-475XL, NorthernLights 493, DY-490, DyLight 488, Alexa Fluor 488, 5-FITC, 5-FAM, DY-495-X5, DY-495, Fluorescein, FITC, ATTO 488, HiLyte Flour 488, MFP 488, ATTO 495, and Oyster 500.

When the uptake amount is higher than 0.068 pmol/2 × 10⁵ cells, the accuracy of predicting *in vivo* pharmacokinetics from *in vitro* pharmacokinetics can be improved. In a preferred aspect, the cellular uptake of the molecule in the present invention can be performed such that the uptake amount is higher than 0.070 pmol/2 × 10⁵ cells, higher than 0.080 pmol/2 × 10⁵ cells, higher than 0.090 pmol/2 × 10⁵ cells, or higher than 0.10 pmol/2 × 10⁵ cells. The upper limit of the uptake amount is not particularly limited, but may be, for example, less than 0.42 pmol/2 × 10⁵ cells, less than 0.40 pmol/2 × 10⁵ cells, less than 0.30 pmol/2 × 10⁵ cells, less than 0.20 pmol/2 × 10⁵ cells, or less than 0.16 pmol/2 × 10⁵ cells. The cellular uptake of the molecule in the present invention may be performed such that the uptake amount may be, for example, higher than 0.068 pmol/2 × 10⁵ cells, preferably higher than 0.070 pmol/2 × 10⁵ cells, higher than 0.080 pmol/2 × 10⁵ cells, higher than 0.090 pmol/2 × 10⁵ cells, or higher than 0.10 pmol/2 × 10⁵ cells, and less than 0.42 pmol/2 × 10⁵ cells, preferably less than 0.40 pmol/2 × 10⁵ cells, less than 0.30 pmol/2 × 10⁵ cells, less than 0.20 pmol/2 × 10⁵ cells, or less than 0.16 pmol/2 × 10⁵ cells.

In one embodiment, the molecule in the present invention is a protein, and step (a) may be performed such that the uptake amount as measured using the fluorescent dye added to the molecule is higher than 0.068 pmol/2 × 10⁵ cells.

In one aspect, step (a) has at least one feature selected from the following (i) to (iii):
(i) a period of the contact of the molecule with the cell is 5 hours or more;
(ii) the cell after the contact with the molecule is not washed under acidic conditions; and
(iii) the cell expresses a target of the molecule on a surface of the cell.

Regarding (i), the period of the contact may be 5 hours or more, e.g., 6, 7, 8, 9, 10, 11, 12, 13, 14, 15, 16, 17, 18, 19, 20, 21, 22, 23, or 24 hours or more. The upper limit of the period of the contact is not particularly limited as long as the molecule in the present invention is stably present and the cellular function such as endocytosis is not lost. The period of the contact may be, for example, 72 hours or less, 48 hours or less, or 36 hours or less. Thus, the contacting period may be 5 hours or more (e.g., 6, 7, 8, 9, 10, 11, 12, 13, 14, 15, 16, 17, 18, 19, 20, 21, 22, 23, or 24 hours or more) and 72 hours or less (e.g., 48 hours or less, or 36 hours or less).

Regarding (ii), the cells may be washed prior to the measurement of *in vitro* pharmacokinetics in step (b) (e.g., when measuring an efflux amount from inside to outside of a cell, an efflux rate from inside to outside of a cell, a rate of molecular decrease in a cell, or a rate of dissociation from FcRn or a target as the *in vitro* pharmacokinetics). When the washing is performed under acidic conditions, the molecule bound to the surface of the cell may be removed. Thus, no washing of the cell under acidic conditions enables the cellular uptake amount of the molecule in the present invention to be increased. The acidic conditions herein refer to less than pH 6.0, e.g., less than or equal to pH 5.5, less than or equal to pH 5.0, less than or equal to pH 4.5, less than or equal to pH 4.0, less than or equal to pH 3.5, or less than or equal to pH 3.0. In this aspect, the period of the contact of the molecule with the cell in the present invention is not particularly limited as long as the molecule in the present invention is stably present and the cellular function such as endocytosis is not lost. The contacting period, for example, may be 5 hours or more, e.g., 6, 7, 8, 9, 10, 11, 12, 13, 14, 15, 16, 17, 18, 19, 20, 21, 22, 23, or 24 hours or more, and 72 hours or less, 48 hours or less, or 36 hours or less.

Regarding (iii), the cell may be either a cell transformed to express a target of the molecule in the present invention on the surface of the cell, or a cell that has not undergone such transformation and expresses an endogenous protein that is a target of the molecule in the present invention on the surface of the cell. The binding of the molecule in the present invention to the target present on the surface of the cell enables the cellular uptake amount of the molecule to be increased. It is more preferred as the expression amount of the target is greater. A sufficient amount of the target can be expressed, for example, by using a promoter commonly used as a promoter for expression in animal cells, such as a promoter such as CMV, PGK, RSV, CAG, EF-1 alpha, SV40, TRE, Oct3/4, or Nanog.

In one aspect, the cellular uptake amount of the molecule in the present invention can be increased when step (a) includes at least one step selected from the following (iv) to (vii):
(iv) adjusting the pH of the aqueous medium to 5.0 to 6.0,
(v) forming, when the molecule is an antibody, an immunoconjugate (IC) between the antibody and its antigen,
(vi) adding an anti-Fc antibody to an aqueous medium when the molecule contains an Fc region; and
(vii) adding an uptake accelerator to the aqueous medium.

Regarding (iv), in an aqueous medium with pH adjusted to 5.0 to 6.0, the molecule in the present invention is positively charged to tend to enter the cell. In addition, in the case of the molecule in the present invention containing an Fc region, the binding capability of the Fc region to FcRn becomes higher under the pH environment in the endosome, which is less than pH 6.5, making the molecule easier to enter the cell. Thus, adjustment of the pH of the aqueous medium to 5.0 to 6.0 may enable the cellular uptake amount of the molecule in the present invention to be increased. Thus, in a preferred embodiment, the molecule in the present invention may contain an Fc region, and step (a) may include step (iv).

Regarding (v), it is known that antibody-antigen complex, i.e., immune complex, tends to be taken up into cells (J Immunol. 1962 Oct;89:471-82, and Int Arch Allergy Appl Immunol. 1974;46(2):230-48). When the molecule in the present invention is an antibody, contacting a cell with an immune complex containing the molecule in the present invention may enable the cellular uptake amount of the molecule to be increased.

Regarding (vi), it is known that a plurality of molecules associates when an anti-Fc antibody binds to an Fc region of the molecule in the present invention, thereby facilitating the uptake of the molecules into cells (J Immunol. 2013 Jun 15; 190 (12): 6694-706). When the molecule in the present invention contains an Fc region, contacting molecules associated by the addition of an anti-Fc antibody with cells may increase the cellular uptake amount of the molecule.

Regarding (vii), examples of the uptake accelerator include an uptake accelerator for proteins. Examples of the uptake accelerator for proteins include BioPORTER(R) protein delivery reagent (Genlantis Inc.), PULSin(R) Kit (Polyplus-transfection(R) SA), Pro-DeliverIN (OZ Biosciences), and L17E Cytosolic Delivery Peptide (PEPTIDE INSTITUTE, INC.).

The uptake accelerator also includes an endocytosis accelerator. Examples of the endocytosis accelerator include ocadic acid (Drug Delivery System 2016, vol. 31, No. 1, p. 83-84).

The uptake accelerator also includes a substance that inhibits the efflux of the molecule in the present invention from cells. Examples of such a substance include inhibitors of ATP-binding cassette transporters (ABC transporters). As the inhibitors of ABC transporters, those commonly used in the art can be used, and examples thereof include MK571, Ceefourin(TM) 1 (manufactured by abcam), Ceefourin(TM) 2 (manufactured by abcam), erythromycin, and thienylbutyl isothiocyanate, which are known as inhibitors of MRP2.

In step (a), a pharmaceutically active component may be further added to the aqueous medium. Then, *in vitro* pharmacokinetics of the molecule in the present invention under the presence of the component can be evaluated. The pharmaceutically active ingredient is not particularly limited as long as it is an agent that can be used in combination with the molecule *in vivo,* and examples thereof include agents for the treatment of diseases such as cancer, autoimmune diseases, infections, neurological diseases, osteoporosis, gonarthrosis/scapulohumeral periarthritis, and hemophilia A.

In the measurement *of in vitro* pharmacokinetics in step (b), an appropriate numerical value is measured depending on the type of *in vitro* pharmacokinetics. The measurement may be performed at a particular time point or may be performed multiple times in a time-dependent manner.

In one embodiment, when the *in vitro* pharmacokinetics is represented by the efflux amount from inside to outside of a cell (Efflux amount), the efflux amount is determined by, after step (a), exchanging the aqueous medium such as culture medium for one that does not contain the molecule in the present invention, and then detecting the molecule in the aqueous medium to measure the efflux amount of the molecule from the cell to outside of the cell.

In one embodiment, when the *in vitro* pharmacokinetics is the efflux rate from inside to outside of a cell (Efflux rate), the efflux rate is determined by, after step (a), exchanging the aqueous medium such as culture medium for one that does not contain the molecule in the present invention, and then detecting the molecule in the aqueous medium to measure the efflux amount of the molecule per unit time.

In one embodiment, when the *in vitro* pharmacokinetics is represented by a rate of molecular decrease in a cell, the rate of molecular decrease in a cell is determined by, after step (a), exchanging the aqueous medium such as culture medium for one that does not contain the molecule in the present invention, and then detecting the molecule in the cell to measure the amount of the molecule decreased from the cell per unit time.

In one embodiment, when the *in vitro* pharmacokinetics is represented by a rate of dissociation from FcRn or a target, the rate of dissociation from FcRn or a target is determined by, after step (a), exchanging the aqueous medium such as culture medium for one that does not contain the molecule in the present invention, and then detecting the molecule in the aqueous medium to measure the efflux amount of the molecule into the aqueous medium per unit time.

Step (b) may be a step of measuring *in vitro* pharmacokinetics at a stage in step (a) of taking up the molecule in the present invention into a cell. Examples of the *in vitro* pharmacokinetics thus measured include a rate of internalization, an amount of transcytosis, a Kp value, and a rate of association with FcRn or a target.

In one embodiment, when the *in vitro* pharmacokinetics is represented by a rate of internalization, the rate of internalization is determined by putting the molecule in the present invention in a state that may provide the cellular uptake of the molecule in step (a), and then measuring the amount of the molecule taken up into the cell from outside of the cell per unit time. In a preferred aspect, the cells contacted with the molecule in the present invention for a predetermined period are washed with an aqueous medium that is acidic (less than pH 6.0, e.g., less than or equal to pH 5.5, less than or equal to pH 5.0, less than or equal to pH 4.5, less than or equal to pH 4.0, less than or equal to pH 3.5, or less than or equal to pH 3.0) prior to the measurement of the amount of the molecule, and the molecule that is bound to the surface of the cell is removed. The rate of internalization can also be determined by measuring the amount of molecules taken up into cells time-dependently after the start of uptake, performing integration plot analysis using the obtained measured values, and calculating a rate of internalization from the initial slope.

In one embodiment, when the *in vitro* pharmacokinetics is represented by an amount of transcytosis, the amount of transcytosis is determined by putting the molecule in the present invention in a state that may provide the cellular uptake of the molecule in step (a), and then measuring the amount of the molecule permeating the cell. For this purpose, cells cultured in a sheet form may be used. Commercially available products that may be used for measurement of the amount of transcytosis (e.g., Transwell(R) permeable support (Corning Incorporated)) may also be used.

In one embodiment, when the *in vitro* pharmacokinetics is represented by a Kp value, the Kp value is determined by putting the molecule in the present invention in a state that provide the cellular uptake of the molecule in step (a), and leaving it for a predetermined period, then measuring the amounts of the molecule in the cell and in the aqueous medium. The Kp value is calculated by (amount in cell)/(amount in aqueous medium).

In one embodiment, when the *in vitro* pharmacokinetics is represented by a rate of association with FcRn or a target, the rate of association with FcRn or a target is determined by putting the molecule in the present invention in a state that may provide the cellular uptake of the molecule in step (a), and then measuring the amount of the molecule binding to FcRn or a target per unit time.

In one aspect, the measuring method of the present invention may further include a step of
(c) calculating an *in vitro* evaluation parameter from a measurement result obtained in step (b).

The "*in vitro* evaluation parameter" as used herein means an index calculated from a numerical value measured as *in vitro* pharmacokinetics. Calculation of *in vitro* evaluation parameters facilitates *in vitro* pharmacokinetic evaluation and *in vivo* pharmacokinetic prediction.

Examples of the *in vitro* evaluation parameter include "clearance index" and "HERA index".

The "clearance index" is calculated by one of the following three methods based on the efflux amount from inside to outside of a cell (Efflux amount).

Method1: The amount of molecules inside of the cells at 0 minutes after the start of efflux and the amount of molecules outside of the cells at 240 minutes after the start of efflux are measured, and the value calculated by (amount of molecules outside of cells at 240 minutes)/(amount of molecules inside of cells at 0 minutes) is taken as the clearance index.

Method2: The amount of molecules inside of the cells at 0 minutes after the start of efflux and the amounts of molecules outside of the cells at 120 and 240 minutes after the start of efflux are measured, and the value calculated by (average value of the amounts of molecules outside of cells at 120 and 240 minutes)/(amount of molecules inside of cells at 0 minutes) is taken as the clearance index.

Method3: The amount of molecules inside of the cells at 0 minutes after the start of efflux and the amounts of molecules outside of the cells at 60, 120, and 240 minutes after the start of efflux are measured, and the value calculated by (average value of the amounts of molecules outside of cells at 60, 120, and 240 minutes)/(amount of molecules inside of cells at 0 minutes) is taken as the clearance index.

In a preferred embodiment, a clearance index according to Methods is calculated as an *in vitro* evaluation parameter.

The "HERA index" is calculated by the following method based on the efflux amount from inside to outside of a cell (Efflux amount).

The cells are allowed to take up the molecule in the present invention by incubating the cells and the molecule in a buffer at pH 6.0 for 4 hours. The cells are then washed and a buffer at pH 7.4 is added for efflux of the molecule from the cells. The efflux amount of the molecule to the buffer (Rx) and the residual amount of the molecule in the cells (RAx) are measured. For a reference molecule (e.g., wild-type protein when the molecule in the present invention is a mutated protein), the efflux amount (Rwt) and the residual amount (RAwt) are also measured in the same way. The value calculated by (Rx/Rwt)/(RAx/RAwt) is taken as HERA score (Non Patent Literature 1).

In a preferred embodiment, the measuring method of the present invention can be a method for measuring *in vitro* pharmacokinetics of an antibody, including steps of:
(a) contacting an antibody with a cell expressing FcRn in an aqueous medium to allow the cell to take up the antibody to be an uptake amount higher than 0.068 pmol/2 × 10⁵ cells, in which step (a) has features of the following (i) to (iii):
   (i) a period of the contact of the antibody with the cell is 24 hours or more;
   (ii) the cell after the contact with the antibody is not washed under acidic conditions; and
   (iii) the cell expresses a target of the antibody on a surface of the cell;
(b) measuring *in vitro* pharmacokinetics of the antibody; and
(c) calculating an *in vitro* evaluation parameter from a measurement result obtained in step (b).
   the antibody contains an FcRn-binding domain and a target-binding domain,
   the *in vitro* pharmacokinetics is an efflux amount from inside to outside of the cell, and
   the *in vitro* evaluation parameter is a clearance index.

The measuring method of the present invention can be used for quality assurance or efficacy prediction of a medicament containing the molecule in the present invention.

In one embodiment, for quality assurance of a medicament, the method of the present invention can be incorporated as a part of the manufacturing process of the medicament, for example, as a standard test of the medicament. The quality of the medicament can be maintained by defining, as a standard, a measurement value *of in vitro* pharmacokinetics or a range to include *in vitro* evaluation parameters, and manufacturing a product that meets the standard.

In one embodiment, efficacy of a medicament can be predicted by measuring *in vitro* pharmacokinetics according to the measuring method of the present invention. For example, in some autoimmune diseases, it is known that auto-antibodies against auto-antigens increase and attack the periphery to induce autoimmune reactions. In an attempt to reduce autoantibodies by inhibiting efflux of intracellularly captured autoantibodies to outside of the cells via FcRn, the use of immunoglobulin formulations (e.g., Heizentra(R) (CSF Behring)) that inject large amounts of human plasma-derived IgG has been reported. It has also been reported that FcRn inhibitors may be developed to treat autoimmune diseases (Folia Pharmacol. Jpn. 136, 280-284 (2010)). Thus, the efficacy of a medicament may be predicted by measuring *in vitro* pharmacokinetics of the molecule of interest by the measuring method of the present invention, and evaluating the binding of the molecule to FcRn based on the measurement.

The efficacy of a medicament may also be predicted by measuring *in vitro* pharmacokinetics by the measuring method of the present invention and predicting *in vivo* pharmacokinetics based on the measurement (see, section II described below).

In certain embodiments, the prediction of efficacy of a medicament may be a prediction of drug-drug interactions. For example, by contacting the molecule in the present invention and another pharmaceutically active component with a cell in step (a) and measuring *in vitro* pharmacokinetics under the presence of the component, it is possible to predict how the component will affect the efficacy of a medicament of the molecule.

### II. Method for predicting in vivo pharmacokinetics of molecule

The second aspect of the present invention relates to a method for predicting *in vivo* pharmacokinetics of a molecule (hereinafter also referred to as the predicting method of the present invention).

The predicting method of the present invention includes steps of:
(a') measuring *in vitro* pharmacokinetics by the measuring method of the present invention; and
(b') predicting *in vivo* pharmacokinetics of the molecule when administered to a living organism, from a measurement value or an *in vitro* evaluation parameter obtained in step (a').

The step (a') is performed according to I described above.

In step (b'), *in vivo* pharmacokinetics is predicted with the measurement values or *in vitro* evaluation parameters obtained in step (a') based on the correlation between the *in vitro* pharmacokinetics measurement values or *in vitro* evaluation parameters and *in vivo* pharmacokinetics calculated in advance. The correlation is determined depending on the types of each molecule, biological species, and *in vitro* and *in vivo* pharmacokinetics, similarly to the specific examples using mice shown below.

Herein, for a reference molecule, shown is an example of predicting a plasma half-life or clearance as *in vivo* pharmacokinetics, in which an efflux amount from inside to outside of a cell is measured as *in vitro* pharmacokinetics, and a clearance index (Methods) is calculated as an *in vitro* evaluation parameter. The reference molecule is selected from a molecule of the same type as the molecule in the present invention (e.g., a protein, a peptide compound, a nucleic acid, a toxin, a virus, or a DDS formulation such as nanoparticles or microparticles) having the same target. For example, when the molecule in the present invention and the reference molecule are antibodies, they bind to the same antigen (preferably the same epitope). When the molecule in the present invention is, for example, an artificial product (e.g., a mutated protein, a mutated peptide compound, or a mutated nucleic acid), the reference molecule may be a molecule used in the production as a reference (e.g., a wild-type protein, a wild-type peptide compound, or a wild-type nucleic acid) and/or another molecule produced in the same manner as in the artificial product. One or more, preferably two or more (e.g., three or more, four or more, five or more, or ten or more) reference molecules are used for determining the correlation.

The *in vitro* pharmacokinetics of the reference molecule is measured by the measuring method of the present invention, as is the molecule in the present invention. As necessary, *in vitro* evaluation parameters are calculated from the measurement results of *in vitro* pharmacokinetics.

For *in vivo* pharmacokinetics, the reference molecule is administered to tail vein of mice expressing FcRn, then plasma antibody concentration is measured time-dependently until 28 days after administration, and then plasma half-life or clearance is calculated by non-compartmental model analysis.

Values *of in vitro* pharmacokinetic measurements or *in vitro* evaluation parameters and values of plasma half-life or clearance are plotted for the reference molecule. A regression line may be created based on the obtained data. A correlation between *in vitro* pharmacokinetics measurement values or *in vitro* evaluation parameters and *in vivo* pharmacokinetics can thus be calculated.

The *in vivo* pharmacokinetics in the predicting method of the present invention is not particularly limited, and examples thereof may include a bioavailability, a volume of distribution, a fraction unbound in blood, a clearance, a urinary excretion rate, a blood concentration half-life, or a mean residence time. In a preferred aspect, the *in vivo* pharmacokinetics is a clearance or a blood concentration half-life, and the *in vitro* evaluation parameter is a clearance index.

In one embodiment, the living organism may be a human, a monkey, a miniature pig, a rat, a mouse, a rabbit, a dog, a guinea pig, a hamster, a chimpanzee, a marmoset, a ferret, or a cat. When the predicting method of the present invention is used for reducing the number of experimental animals used, the living organism may be a non-human animal such as a monkey, a miniature pig, a rat, a mouse, a rabbit, a dog, or a guinea pig.

According to the predicting method of the present invention, *in vivo* pharmacokinetics such as plasma half-life or clearance can be predicted by *in vitro* testing. The predicting method of the present invention can thus be used as an alternative to *in vivo* pharmacokinetic testing with animals. As a result, the number of *in vivo* pharmacokinetic testing and the number of experimental animals used can be reduced, and thus the present invention is also useful from the viewpoint of animal ethics.

### III. Method for screening molecule

A third aspect of the present invention relates to a method for screening a molecule (hereinafter referred to as the screening method of the present invention).

The screening method of the present invention includes steps of:
(a") providing two or more different molecules that bind to the same target,
(b") measuring *in vitro* pharmacokinetics of each of the two or more molecules provided in step (a") by the measuring method of the present invention; and
(c") comparing the measurement values or *in vitro* evaluation parameters for each of the two or more molecules obtained in step (b") with each other to select a molecule indicating a desired value.

The two or more molecules in step (a") each are the molecule in the present invention described in I above. The two or more molecules are the same type of molecule and have the same target, but are different molecules from each other. For example, when the two or more molecules are antibodies, they may be modified antibodies derived from the same parent antibody but having different modifications from each other.

Step (b") is performed for each of the two or more molecules according to I described above.

In step (c"), a molecule indicating a desired measurement value of *in vitro* pharmacokinetics or a desired value of *in vitro* evaluation parameter is selected. The desired value may vary depending on the type *of in vitro* pharmacokinetics, but may be, for example, a value indicating a higher binding activity to FcRn or the target. When the *in vitro* pharmacokinetics is an efflux amount from inside to outside of a cell, an efflux rate from inside to outside of a cell, an amount of transcytosis, or a rate of molecular decrease in a cell, the higher value indicates a higher binding activity to FcRn. When the *in vitro* pharmacokinetics is a rate of internalization and when the cell expresses the target, the higher value indicates a higher binding activity to the target. Each molecules selected can be used for applications according to its characteristics (such as medicaments) and may be subjected to further testing.

According to the screening method of the present invention, a molecule having desired characteristics can be selected without performing an *in vivo* pharmacokinetic test. As a result, the number of *in vivo* pharmacokinetic testing and the number of experimental animals used can be reduced, and thus the present invention is also useful from the viewpoint of animal ethics.

All references cited herein are incorporated herein by reference.

The present invention will be described in more detail by way of Examples.

### EXAMPLES

### Example 1 Mouse plasma pharmacokinetic evaluation of each Fc-modified antibody

### (1-1) Characteristics of Fc region of antibody used for uptake evaluation

H237-G1d, H237-F1847m, H237-F1886m, H237-F1927m, and H237-F890, which are anti-IL-6R antibodies having Fc described in WO 2012/133782 A1, WO 2013/046704 A2, WO 2017/046994 A1, and WO 2009/125825 A1, were used.

The heavy chain sequence of H237-G1d is the amino acid sequence of SEQ ID NO: 79 in WO 2012/133782 A1.

The heavy chain sequence of H237-F1847m is the amino acid sequence of SEQ ID NO: 50 in WO 2017/046994 A1.

The heavy chain sequence of H237-F1886m is the amino acid sequence of SEQ ID NO: 52 in WO 2017/046994 A1.

The heavy chain sequence of H237-F1927m is the amino acid sequence of SEQ ID NO: 54 in WO 2017/046994 A1.

The heavy chain sequence of H237-F890 is the amino acid sequence of SEQ ID NO: 6 in WO 2013/046704 A2.

The light chain sequences of all of these are the amino acid sequence of SEQ ID NO: 27 in WO 2009/125825 A1.

### (1-2) Plasma pharmacokinetic evaluation in mice

To mouse FcRn knockout/human FcRn transgenic mice (Tg#32, male), 1 mg/kg of any one of H237-G1d, H237-F1847m, H237-F1886m, H237-F1927m, and H237-F890 antibodies and 1000 mg/kg of Sanglopor (dried pH4 treated human immunoglobulin, CSL Behring) were administered by tail vein. From 5 minutes to 28 days after the administration, jugular venous blood sampling was performed time-dependently. The resulting blood was centrifuged (12000 rpm, 4°C, 5 min) to obtain plasma. Plasma antibody concentration was measured by electrochemical luminescence immunoassay (ECL) using capture and detection antibodies against the administered antibody. In addition, non-compartment model analysis was performed using the obtained PK profile to calculate a half-life and a clearance.

The results are shown in Figure 1. H237-F1847m, H237-F1886m, and H237-F1927m had a gradual slope in the elimination phase compared to H237-G1d and H237-F890, showing a more gradual tendency to disappear from plasma. The calculated PK parameters are shown in Table 1. The H237-F1886m had the longest half-life in the terminal phase, and H237-F1927m and H237-F1847m also had a longer half-life than H237-G1d. H237-F890 had the shortest half-life. The clearance of H237-F1886m was the smallest, and those of H237-F1927m and H237-F1886m were also smaller than that of H237-G1d.

**[Table 1]**

| Table 1: Plasma half-life and clearance of each antibody | | |
|---|---|---|
| | Half-life (day) | Clearance (mL/day/kg) |
| G1d | 12.3 | 9.50 |
| F1847m | 17.2 | 6.02 |
| F1886m | 22.2 | 4.05 |
| F1927m | 19.6 | 5.16 |
| F890 | 8.54 | 8.44 |

### Example 2 Comparison of cellular uptake of each Fc modified antibody

### (2-1) Alexa647 labeling of Fc region-modified antibodies

H237-G1d, H237-F1847m, H237-F1886m, H237-F1927m, and H237-F890 were labeled with Alexa 647 (AF647) using the Alexa flour 647 labeling kit (Thermo Fisher Scientific) according to the attached protocol. The concentration of each antibody and the labeling efficiency of the fluorescent material were measured for absorbance by Nanodrop (Thermo Fisher Scientific) and calculated according to the calculation formula described in the attached protocol.

### (2-2) Evaluation of uptake of Fc region-modified antibodies into hFcRn-hIL6R-CHO cells and hFcRn-CHO cells

To a 50 µL cell solution containing 2 × 10⁵ CHO cells in which human FcRn and human IL-6R were over-expressed (hFcRn-hIL6R-CHO cells (Chiome Bioscience Inc.), which were produced by introducing expression vectors containing CMV promoters (pcDNA3.1 vector, Invitrogen) into CHO cells) or CHO cells in which only human FcRn was over-expressed (hFcRn-CHO cells (Chiome Bioscience Inc.), which were produced by introducing expression vectors containing CMV promoters (pcDNA3.1 vector, Invitrogen) into CHO cells) in complete medium (CHO-S-SFM II (Invitrogen)), AF647-labeled antibodies were added to be a final concentration of 50 µg/mL and set to 100 µL/well in a 96-well plate. Thereafter, the reaction was carried out in a CO₂ incubator at 37°C for 24 hours. The cells were then ice-cooled and washed with cold 2% FBS-containing PBS (FBS-PBS). The fluorescence intensity of the cells was measured using FACS CantoII (Becton, Dickinson and Company).

In addition to the cells assayed, the fluorescence intensity of the fluorescently labeled preparation beads was also measured using Quantum MESF (Bangs Laboratories, Inc.) according to the attached protocol. According to the attached protocol, a calibration curve was drawn from the geometric mean fluorescence intensity of each preparation, and the uptake amount of each antibody was calculated from the geometric mean fluorescence intensity of the sample allowed to take up each antibody.

The results are shown in Figure 2. The uptake amount of each antibody was increased in hFcRn-hIL6R-CHO cells with the range of 2.2 to 36-fold compared to hFcRn-CHO cells.

### Example 3 Time-dependent evaluation of cellular uptake of each Fc-modified antibody

To a 50 µL cell solution containing 2 × 10⁵ hFcRn-hII,6R-CHO cells, AF647-labeled antibodies were added to be a final concentration of 50 µg/mL and set to 100 µL/well in a 96-well plate. The reaction was then allowed to proceed while stirring the plates at 37°C for up to 24 hours in a time-dependent manner. After that, the cells were ice-cooled, cold 2% FBS-containing PBS was added, and the cells were washed once with FBS-PBS or a culture medium adjusted to pH 3.0 (acid). The cells were then collected by centrifugation (1000 g, 3 min). The fluorescence intensity of the cells was measured using FACS CantoII.

In addition to the cells assayed, the fluorescence intensity of the fluorescently labeled preparation beads was also measured using Quantum MESF (Bangs Laboratories, Inc.) according to the attached protocol. According to the attached protocol, a calibration curve was drawn from the geometric mean fluorescence intensity of each preparation, and the uptake amount of each antibody was calculated from the geometric mean fluorescence intensity of the sample allowed to take up each antibody.

The results are shown in Figure 3(a) and 3(b). For each antibody, time-dependent increase of the uptake amount was observed. H237-F890 and H237-F1886m showed slightly higher uptake amount compared to H237-G1d, H237-F1847m, and H237-F1927m in both cases of FBS-PBS washing and Acid washing.

Integration plot analysis was performed using the obtained measurement value of the antibody uptake amount, and the rate of internalization was calculated from the initial slope. The results of the Integration plot analysis are shown in Figure 3(c). The calculated rate of internalization is shown in Table 2. H237-F890 showed slightly higher values than H237-G1d, H237-F1847m, H237-F1886m, and H237-F1927m.

**[Table 2]**

| Table 2: Rate of internalization (kint) of each antibody | |
|---|---|
| | kint (1/day) |
| G1d | 20.5 |
| F1847m | 19.1 |
| F1886m | 24.0 |
| F1927m | 32.5 |
| F890 | 63.0 |

### Example 4 Time-dependent evaluation of each Fc-modified antibody on intracellular antibody amount and efflux amount into culture medium

### (4-1) Time-dependent evaluation of intracellular antibody amount and efflux amount into culture medium

To a 50 µL cell solution containing 2 × 10⁵ hFcRn-hII,6R-CHO cells, AF647-labeled antibodies were added to be a final concentration of 50 µg/mL and set to 100 µL/well in a 96-well plate. The cells were then incubated at 37°C for 24 hours. After that, the cells were ice-cooled, cold 2% BSA-containing culture medium was added and removed, and 100 µL of fresh 2% BSA-containing culture medium was added. Reaction was performed at 37°C for up to 4 hours, and sampling was performed time-dependently. Samples at each time point were centrifuged, supernatants were collected, and the cells were washed with FBS-PBS. The antibody concentration in the supernatant was measured using electrochemical luminescence immunoassay (ECL). The fluorescence intensity of the cells was measured using FACS CantoII, and the amount of antibodies contained in the cells was calculated based on the fluorescence intensity of the preparation beads.

The results are shown in Figure 4(a). For all antibodies, a time-dependent decrease of intracellular antibody amount was observed. The intracellular antibody amount of H237-F890 was shifted within a high level.

In addition, the profile of the efflux amount of antibody into the culture medium versus time is shown in Figure 4(b). It was shown that the all antibodies were rapidly effluxed until about 30 minutes after starting of the efflux, then reached the plateau. Up to 10 minutes after the start of efflux, graphs for all antibodies showed a similar slope, but after 60 minutes, differences among the antibodies were seen in the amount of antibodies in the culture medium, with H237-F1886m being the most and H237-G1d the least.

### (4-2) Calculation of clearance index

The clearance index was calculated with the following three calculation formulas using the intracellular antibody amount at 0 minutes after the start of efflux and the efflux amount of antibody into the culture medium until each time point.
Method1: (antibody amount in culture medium at 240 minutes)/(intracellular antibody amount at 0 minutes)
Method2: (average antibody amount in culture medium at 120 and 240 minutes)/(intracellular antibody amount at 0 minutes)
Method3: (antibody amount in culture medium at 60, 120, and 240 minutes)/(intracellular antibody amount at 0 minutes)

Table 4 shows the value of clearance index calculated by the methods above. For all antibodies, the values ranged from about 0.30 to 0.70. The values calculated for each antibody by the three methods showed roughly the same value. The degree of differences in the values between the antibodies also showed the same trend in the three methods.

**[Table 3]**

| Table 3: Clearance index of each antibody | | | | | |
|---|---|---|---|---|---|
| | G1d | F1847m | F1886m | F1927m | F890 |
| Method1 | 0.46 | 0.49 | 0.71 | 0.56 | 0.28 |
| Method2 | 0.45 | 0.50 | 0.71 | 0.56 | 0.39 |
| Methods | 0.49 | 0.63 | 0.73 | 0.62 | 0.41 |

### Example 5 Correlation between clearance index and in vivo pharmacokinetics

The correlation between the clearance index calculated in Example 4 and the *in vivo* half-life or clearance measured in Example 1 was evaluated. The results are shown in Figure 5. Both the *in vivo* half-life and clearance were strongly correlated with the clearance index (R² = 0.961 and R² = 0.822, respectively). Thus, it was shown that the *in vivo* plasma half-life or clearance is predictable by the clearance indexes calculated by Methods 1 to 3.

### INDUSTRIAL APPLICABILITY

According to the present invention, *in vivo* pharmacokinetics of a large number of medicament candidates can be predicted with greater simplicity and higher accuracy than conventional methods. The present invention can also contribute to the reduction of the number of experimental animals used and to the development of more pharmacologically effective medicaments.

## Claims

1. A method for measuring *in vitro* pharmacokinetics of a molecule, the method comprising steps of:
(a) contacting a molecule with a cell expressing FcRn in an aqueous medium to allow the cell to take up the molecule to be an uptake amount higher than 0.068 pmol/2 × 10⁵ cells, in which step (a) has at least one feature selected from the following (i) to (iii):
(i) a period of the contact of the molecule with the cell is 5 hours or more;
(ii) the cell after the contact with the molecule is not washed under acidic conditions; and
(iii) the cell expresses a target of the molecule on a surface of the cell; and
(b) measuring *in vitro* pharmacokinetics of the molecule,
in which the molecule contains an FcRn-binding domain.

2. The method according to claim 1, wherein the molecule is an antibody containing an FcRn-binding domain and a target-binding domain.

3. The method according to claim 1 or 2, wherein the cell is a cell transformed to express FcRn.

4. The method according to any one of claims 1 to 3, wherein the cell is a cell transformed to express a target of the molecule on a surface of the cell.

5. The method according to claim 3 or 4, wherein the cell is a CHO cell, a HEK293 cell, a COS-1 cell, a COS-7 cell, an MDCK cell, an HMEC1 cell, a HELA cell, a HepG2 cell, or a BaF cell.

6. The method according to claim 1 or 2, wherein the cell is a liver parenchymal cell, a liver non-parenchymal cell, a hepatic sinus endothelial cell, a Kupffer cell, a human umbilical vein endothelial cell, a peripheral blood mononuclear cell PBMC, a macrophage, a mononuclear cell, a B cell, a T cell, a platelet, an NK cell, a neutrophil, an eosinophil, a basophil, a granulocyte, or a dendritic cell.

7. The method according to any one of claims 1 to 6, wherein the cell is allowed to take up the molecule to be an uptake amount higher than 0.10 pmol/2 × 10⁵ cells.

8. The method according to any one of claims 1 to 7, wherein the *in vitro* pharmacokinetics is an efflux amount from inside of a cell to a culture medium, an efflux rate from inside of a cell to a culture medium, a rate of internalization, an amount of transcytosis, a Kp value, a rate of molecular decrease in a cell, a rate of association with FcRn or a target, or a rate of dissociation from FcRn or a target.

9. The method according to any one of claims 1 to 8, wherein FcRn is human FcRn, monkey FcRn, miniature pig FcRn, rat FcRn, mouse FcRn, rabbit FcRn, dog FcRn, or guinea pig FcRn.

10. The method according to any one of claims 1 to 9, further comprising a step of (c) calculating an *in vitro* evaluation parameter from a measurement result obtained in step (b).

11. The method according to claim 10, wherein the *in vitro* evaluation parameter is a clearance index or a HERA score.

12. The method according to any one of claims 1 to 11, wherein the method is used for quality assurance or efficacy prediction of a medicament containing the molecule.

13. The method according to any one of claims 1 to 12, wherein the target of the molecule is a membrane protein.

14. The method according to claim 13, wherein the target of the molecule is a human IL6 receptor.

15. A method for predicting *in vivo* pharmacokinetics of a molecule, the method comprising:
(a') measuring *in vitro* pharmacokinetics by the method according to any one of claims 1 to 14; and
(b') predicting *in vivo* pharmacokinetics of the molecule when administered to a living organism, from a measurement value or an *in vitro* evaluation parameter obtained in step (a').

16. The method according to claim 15, wherein the *in vivo* pharmacokinetics is a bioavailability, a volume of distribution, a fraction unbound in blood, a clearance, a urinary excretion rate, a blood concentration half-life, or a mean residence time.

17. The method according to claim 15 or 16, wherein the living organism is a human, a monkey, a miniature pig, a rat, a mouse, a rabbit, a dog, or a guinea pig.

18. The method according to any one of claims 15 to 17, which is used as an alternative to a pharmacokinetic test method using an animal.

19. A method for screening a molecule, the method comprising:
(a") providing two or more different molecules that bind to the same target,
(b") measuring *in vitro* pharmacokinetics of each of the two or more molecules provided in step (a") by the method according to any one of claims 1 to 14; and
(c") comparing the measurement values or *in vitro* evaluation parameters for each of the two or more molecules obtained in step (b") with each other to select a molecule indicating a desired value.
